# EUROPEAN PATENT APPLICATION

(11) **EP 2 338 525 A2**
(43) Date of publication of application: **29.06.2011**
(21) Application number: 11159219.2
(22) Date of filing: 09.07.2004
(51) Int. Cl.: A61K 49/18, A61K 49/12, A61K 49/00, A61K 47/48, A61K 9/127, A61P 35/00

(54) **Remote detection of substance delivery to cells**

(30) Priority: 09.07.2003 US 486080 P
(62) Divisional of application: 04777914.5
(71) Applicant: California Pacific Medical Center, San Francisco, CA 94107 (US)
(72) Inventor: Drummond, Daryl, C., Concord, CA 94519 (US); Hong, Keelung, San Francisco, CA 94107 (US); Kirpotin, Dmitri, B., San Francisco, CA 94121 (US)
(74) Representative: Sutcliffe, Nicholas Robert

(57) **Abstract**

The present invention provides for the development of endocytosis-sensitive probes, and a remote method for measuring cellular endocytosis. These probes are based on the reduced water permeability of a nanoparticle or liposomal delivery system, and inherent degradability or disruption of barrier integrity upon endocytosis. The invention also provides for liposomes having combined therapeutic and diagnostic utilities by co-encapsulating ionically coupled diagnostic and therapeutic agents, in one embodiment, by a method using anionic chelators to prepare electrochemical gradients for loading of amphipathic therapeutic bases into liposomes already encapsulating an imaging agent. The invention provides for imaging of therapeutic liposomes by inserting a lipopolymer anchored, remotely sensing reporter molecules into liposomal lipid layer. The invention allows for an integrated delivery system capable of imaging molecular fingerprints in diseased tissues, treatment, and treatment monitoring.

## Description

### Statement Regarding Federally Sponsored Research

The U.S. Government has a paid-up license in this invention and the right in limited circumstances to require the patent owner to license others on reasonable terms as provided for by the terms of contract number N01-CO-27176 awarded by the National Cancer Institute.

### Cross-Reference to Other Applications

This application claims priority to U.S. Provisional Application No. 60/486,080 filed July 9, 2003, and incorporates the entire contents of that provisional application herein by reference.

### INTRODUCTION

### Field of the Invention

The present invention provides for the development of endocytosis-sensitive probes, and a remote method for measuring cellular endocytosis. The invention also provides for liposomes having combined therapeutic and diagnostic utilities. The invention additionally provides for imaging of therapeutic liposomes. Further, the invention allows for an integrated delivery system capable of imaging molecular fingerprints in diseased tissues, treatment, and treatment monitoring.

### Background of the Invention

Liposomes containing paramagnetic ions have been investigated as contrast agents for Magnetic Resonance (MR) imaging. Early results were hindered by obstacles such as poor encapsulation efficiency of paramagnetic ions, liposome instability, toxicity of certain encapsulated agents and poor relaxivity (Caride, et al., Magn Reson Imaging, 2: 107-112, 1984; Magin et al., Magn Reson Med, 3: 440-447, 1986; Navon et al., Magn Reson Med, 3: 876-880, 1986; Unger et al., Invest Radiol, 20: 693-700, 1985). Relaxivity is the property whereby an MR contrast medium alters the relaxation times of the material it acts on. At the concentrations normally used in MR imaging, the effect of an MR contrast medium predominantly acting on T1 relaxation augments the initial relaxation rate which is proportional to the concentration of the MR contrast medium. As a result, if T10 is the initial T1 relaxation time and r1 is the relaxivity, the T1 relaxation time in the presence of the agent is (1/T1) = (1/T10) + r1C where C is the concentration of the MR contrast medium. Improved relaxivity results in improved contrast of the image obtained by MR imaging techniques.

One of the first approaches taken was the encapsulation of a MR contrast agent, such as ferrite particles or paramagnetic compounds, within the aqueous space of the liposome. An advantage of encapsulation is a decrease in the toxicity of MR contrast agents such as ferrite particles or free manganese. Early *in vitro* MR studies of liposomes encapsulating the clinically approved contrast agent gadolinium diethylenetriamine pentaacetic acid (GdDTPA) showed a decrease in relaxivity as compared to the free agent as a result of shielding of paramagnetic centers from surrounding water (Unger et al., Invest Radiol, 23: 928-932, 1988). However, it was also shown that liposomes with diameters <100 nm demonstrated greater relaxivity than those of larger diameter (Unger et al., MR imaging. Radiology, 171: 81-85, 1989). This is due to the higher flux of water across the liposomal membrane, which can be attributed to the greater surface area:volume ratio in smaller liposomes. The relaxivity of Gd-DTPA entrapped in small unilamellar liposomes has been found to vary with 1/r, where r is the radius of the liposomal membrane (Tilcock et al., Biochim Biophys Acta, 1022: 181-186, 1990). However, a limitation of using liposomes with increasingly smaller diameters is that the amount of encapsulated agent decreases compared to the amount of lipid in the bilayer. It was also shown that liposomes containing cholesterol, had a lower relaxivity than liposomes without cholesterol. Another approach is the conjugation of paramagnetic agents to the surface of the liposome. Paramagnetic polymerized liposomes (PPLs) have been synthesized from a type of polymerizable lipid molecule that has a derivative of gadopentetate dimeglumine as the hydrophilic headgroup and diacetylene groups in the hydrophobic acyl chains, which cross-link when irradiated with ultraviolet light (Storrs et al., J Magn Reson Imaging, 5: 719-724, 1995). MR studies of Lewis rats injected with PPLs (0.015 mmol/kg Gd³⁺) showed enhancement in the kidney and liver consistent with a prolonged blood half-life as compared to conventional liposomes. This was confirmed by studies of ¹¹¹In-labeled PPLs for which a blood pool half-life of 19 h was calculated. In a later study, PPLs were conjugated with biotinylated antibodies against αᵥβ₃, the endothelial integrin, which has been shown to correlate with tumor grade. These immunoliposomes were injected into tumor bearing rabbits which then showed enhanced tumor visualization on images acquired 24 h post-injection. In some images 'hot spots' of tumor angiogenesis could be detected (Sipkins et al., Nat Med, 4: 623-626, 1998).

### Summary of the Invention

One embodiment of the invention provides a liposome for remote sensing of endocytosis for example, by pathological cells, useful in diagnosis of disease by non-invasive methods (MRI, radionuclide, intravital optical imaging). The liposome may comprise a lipopolymer conjugated to a detectable marker, for example, a paramagnetic metal (e.g. gadolinium) chelate, whose signal is modulated upon endocytosis of the liposome into a cell of interest. In a preferred embodiment, liposomes comprising gadolinium DTPA-BMA, gadolinium DTPA, or gadolinium HP-DO3A complexes encapsulated within poorly water-permeable membrane have low effect on proton relaxivity. Upon endocytosis, the liposome is degraded releasing the chelate and concomitantly increasing the effect on proton relaxivity which is remotely detected by MRI method. Alternatively, a liposome is made with encapsulated fluorescent marker in the presence of a fluorescent quencher, or under self-quenching concentrations. Endocytosis and consequent lysosomal degradation of the liposome causes the release of the marker which is accompanied by increase of fluorescence intravitally detected by laser imaging methods. The detection is made specific to a cell comprising a surface marker molecule by incorporation of a cell-internalizable ligand, such as an antibody, specific to the marker. To improve the endocytosis-specific signal modulation, the liposome is sensitized to intracellular conditions, such as decreased pH, for example, by incorporation of pH-sensitive lipopolymers.

The invention also provides a method for remote sensing of endocytosis of a liposome, which method comprises (a) contacting a cell with a liposome comprising a lipopolymer conjugated to a detectable marker whose signal is modulated upon endocytosis of the liposome into a cell of interest under conditions in which endocytosis can occur, and (b) detecting the signal of the detectable marker after endocytosis. Preferably, the detectable marker is a paramagnetic gadolinium chelate comprising gadolinium DTPA-BMA, gadolinium DTPA, or gadolinium HP-DO3A. A preferred embodiment of the invention comprises detecting endocytosis of a liposome encapsulating a fluorescent marker and a fluorescent quencher using laser imaging methods.

Another embodiment of the present invention provides a liposome combining a therapeutic agent and a remotely detectable marker. The liposome preferably comprises a therapeutic agent and a remotely detectable marker conjugated to a lipopolymer. The therapeutic agent is incubated with the conjugated lipopolymer under the conditions providing for stable association of the lipopolymer-marker conjugate with the liposome. A preferred embodiment includes a chelating lipopolymer conjugate, such as, distearoylamino-PEG-(Lys-Gd-DOTA)₄. Upon incubation with agent-loaded liposomes, the lipopolymer anchors itself in the liposomal membrane, providing for non-invasive monitoring of the liposomal drug in a patient's body. In a preferred embodiment, the therapeutic agent is an anti-cancer agent, such as doxorubicin.

In yet another embodiment, the invention provides improved liposomes co-encapsulating a therapeutic agent and a remotely detectable marker. The therapeutic agent and detectable marker are members of an ionically coupled pair, that is, if one is an anion, the other is a cation, and vice versa. A member of the pair is encapsulated in the liposome in a form providing for transmembrane ionic, chemical, or electrochemical gradient that enhances the encapsulation of the other member through a highly efficient "remote loading" principle. Optionally, two members form a stable ionic complex, or salt, that also improves the drug encapsulation stability and/or drug efficacy. An exemplary embodiment includes a liposome with encapsulated anionic MRI marker, Gd-DTPA, in the form of a diammonium salt. Upon removal of extraliposomal marker, transmembrane gradient of ammonium ion is created that affords highly efficient (e.g. >95%) co-loading of a cationic anticancer drug, such as doxorubicin, in the same liposome. The resulting dual-loaded liposome allows non-invasive monitoring of the therapeutic agent in a patient.

Another aspect of the invention is a method for non-invasive monitoring of a liposomal drug in a patient's body. The method comprises: (a) administering a liposome comprising (i) a remotely detectable marker whose signal is modulated upon endocytosis of the liposome into a cell of interest, wherein said remotely detectable marker is conjugated to a lipopolymer and (ii) a therapeutic agent and (b) detecting the signal of the remotely detectable marker.

Other aspects of the invention will be apparent to one of skill in the art upon reading the ensuing specification and claims.

### Brief Description of the Figures

FIG. 1 illustrates how the exchange of water across liposomal membranes is remarkably sensitive to the phase transition of the lipids that compose those membranes.

FIG. 2 depicts the release of a Gd³⁺-contrast agent following disruption of liposomal membrane. Release of the chelate or even simple breakdown of the membrane barrier for a more rapid exchange of water results in a dramatic increase in the relaxivity (R) for the agent.

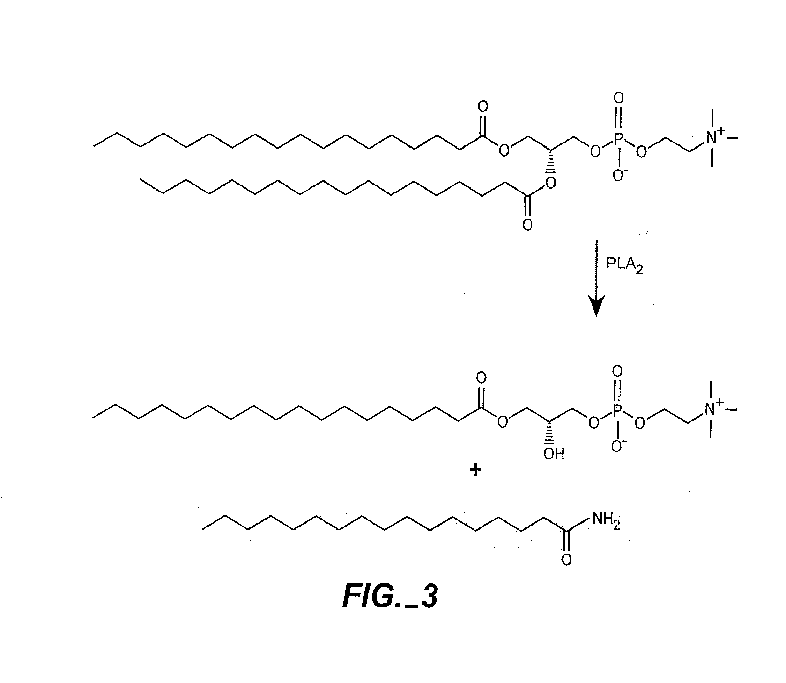

FIG. 3 depicts the action of phospholipases to degrade phospholipids by hydrolyzing one of its four ester bonds; phospholipase A2 cleaves at the sn-2 position, phospholipase C at the glycerol side of the phosphate, and phospholipase D at the head group side of the phosphate ester.

FIG. 4 depicts the chemical structure of poly(NIPAM-co-MAA-co-DODA).

FIG. 5 provides a schematic illustration of the mechanism for pH-triggered release by co-polymers of NIPAM.

FIG. 6 provides a scheme demonstrating the enzyme sensitivity of a glucuronate-quenched Gd³⁺ MRI agent (Scheme 1).

FIG. 7 provides a synthesis scheme for a pH-sensitive DOTA chelator for Gd³⁺ using an acid-hydrolyzable citraconyl linker (Scheme 2).

FIG. 8 illustrates the effect of incorporation of DODA-poly(NIPA-co-MAA) into DSPC/Chol/PEG-DSPE (3:2:0.015) liposomes during the extrusion process on liposomal contents release at different pHs. Liposomes were incubated at various pHs for 5 min at 37 °C and subsequently analyzed on a fluorimeter for HPTS at 520 nm following excitation at 316 nm.

FIG. 9 illustrates the effect of incorporation of DODA-poly(NIPA-co-MAA) into DSPC/Chol/PEG-DSPE (3:2:0.015) liposomes by insertion on liposomal contents release at different pHs. Liposomes were incubated at various pHs for 5 min at 37°C and subsequently analyzed on a fluorimeter for HPTS at 520 nm following excitation at 316 nm.

FIG. 10 provides a synthesis scheme for bis-carboxymethyl-PEG mono-(N,N-dioctadecyl)amide tetra(carbobenzoxy-L-lysine) conjugate (DSA-PEG-(Z-Lys)₄) (Scheme 3).

FIG. 11 provides a synthesis scheme for N-DOTA-poly(ethylene glycol)-dioctadecylamine (Scheme 4).

FIG. 12 provides a synthesis scheme for cholesterol hemisuccinatyl-poly(ethylene) glycol (Scheme 5).

FIG. 13 provides a synthesis scheme for 1,2-distearoyl-sn-glycero-3-phosphoethanol-N-aminoglutaric acid (Scheme 6).

FIG. 14 provides a synthesis scheme for O-glutaryl-dioctadecylglycerol (glutaryl-DOG) (Scheme 7).

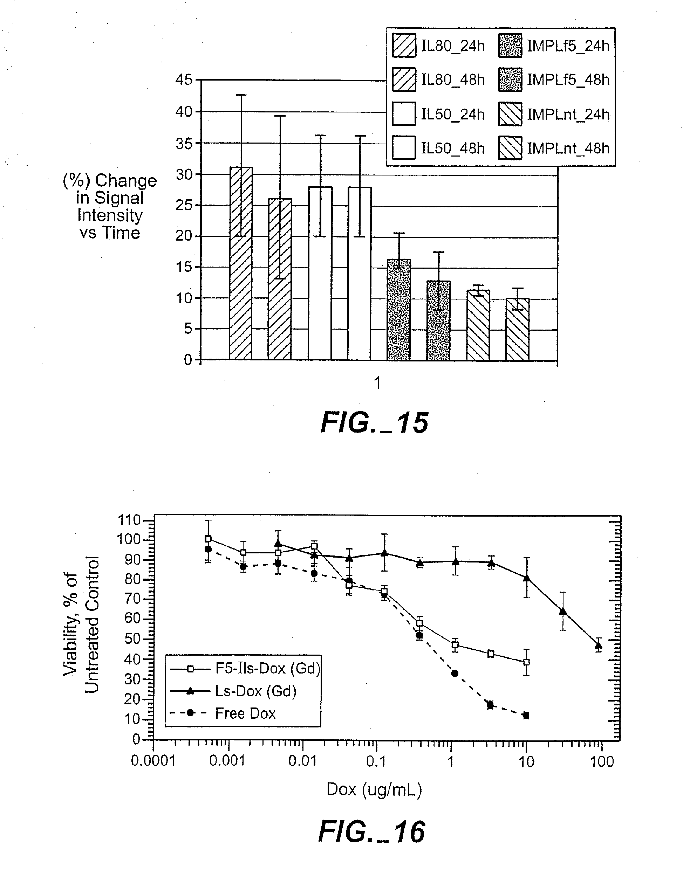

FIG. 15 provides a graphic representation of the changes of MRI signal intensity in BT-474 xenograft tumors after intravenous administration of liposomes containing Gadolinium chelate (Gd-DTPA-BMA) in mice. IL80: "water-permeable" DOPC/Chol/PEG-DPSE (3:1:1) anti-HER2 targeted immunoliposomes. 80 nm in size; IL50: "water-permeable" DOPC/Chol/PEG-DPSE (3:1:1) anti-HER2 targeted immunoliposomes, 50 nm in size; IMPLf5: "water-impermeable" DSPC/DPPC/Chol/PEG-DPSE (2.2:0.3:2:0.3) anti-HER2 targeted immunoliposomes, approx. 75 nm in size; IMPLnt: "water-impermeable" DSPC/DPPC/Chol/PEG-DPSE (2.2:0.3:2:0.3) non-targeted immunoliposomes, approx. 75 nm in size.

FIG 16 is a graph demonstrating in vitro cytotoxicity of free doxorubicin ("DOX"), F5scFv-targeted immunoliposomal doxorubicin, or non-targeted liposomal doxorubicin co-encapsulated with Gd-DTPA against HER2-overexpressing SKBR3 cells.

FIG 17 is a graph demnostrating plasma stability of doxorubicin encapsulation in liposomes loaded using TEA-Gd-DTPA as a loading-assisting gradient-forming component for the drug. The change in doxorubicin to phospholipid ratio is indicative of the loss of doxorubicin from the liposome. The liposome samples were incubated with 50 % human plasma in a microdialysis assay at 37°C.

FIG 18 is a graph demonstrating plasma stability of gadolinium encapsulation in liposomes loaded using TEA-Gd-DTPA as a loading-assisting gradient-forming component for the drug. The amount of Gd is determined by the proton relaxivity measurements followed dissociation of the liposomes by a detergent (Triton X-100). The change in Gd to phospholipid ratio is indicative of the loss of Gd from the liposome. The liposome samples were incubated with 50 % human plasma in a microdialysis assay at 37°C ..

### DETAILED DESCRIPTION

Remotely detectable probes, such as, for example, paramagnetic or supramagnetic MRI probes, can be encapsulated in the liposome interior in a non-signaling ("off") configuration to enable signal generation ("on") upon tumor cell binding and internalization. Internalization can be a result of non-specific or specific, receptor-mediated endocytosis. Targeted probe delivery based on ligand-receptor recognition and tumor cell internalization provides a powerful strategy for detecting important and functional molecular characteristics of cancer cells. These modifications can be readily combined within a multifunctional immunoliposome reporter having both surface-linked radionuclides and interior-encapsulated MRI agents. Because this immunoliposome reporter is based upon the same modular structure and targeting technology as immunoliposomal drugs, the two can be co-developed for use in a closely integrated strategy for imaging, treatment, and treatment monitoring.

The invention provides for diagnostic liposomes capable of remote signaling upon internalization and release in target cells. This approach affords remote sensing of endocytosis dependent, for example, on the presence of a pathologic marker on the cells within the patient's body, for which purpose the liposome with endocytosis-triggered detectable signal is combined with ligand-directed targeting for intracellular delivery. This method is helpful in detecting a diseased condition of the body in a non-invasive way.

The invention also provides for the liposomes having combined therapeutic and diagnostic utility. In one preferred embodiment, a liposome is loaded with a therapeutic agent. Then, the liposome is contacted with a lipopolymer conjugated to remotely detectable functionality under conditions permitting the lipopolymer conjugate to become stably associated with the liposome. One example of a therapeutic agent is a cytotoxic anticancer drug or gene, and a diagnostic compound is a remotely detectable lipopolymer conjugate such as a lipid-poly(ethylene glycol)-chelator conjugate complexed with a paramagnetic ion, such as Gadolinium(III), or a radioactive ion, such as Gallium-67 or Indium-111. Such exemplary liposomes will therefore allow a physician to monitor the delivery of treatment into a patient's tumor by non-invasive radioactivity scanning or magnetic resonance imaging (MRI) and consequently, to optimize the treatment strategy for each patient. Preferably, the diagnostic compound, with remote sensing properties, and the therapeutic compound are members of an ionically matched pair, that is, if one is an anion, the other is a cation, and vice versa. One member of such ionically matched pair is first loaded into a liposome in such a way as to create a transmembrane ionic or electrochemical gradient capable of assisting the subsequent loading of a second member by the "active loading" principle. For example, a remote-sensing compound with MRI contrast properties can be diammonium gadolinium diethylenetriaminepentaacetate (NH₄)₂Gd-DTPA. When entrapped into a liposome and further removed from the extraliposomal medium, this compound also provides for ammonium ion gradient that causes highly effective (practically quantitative) co-loading of a cationic anticancer drug doxorubicin that forms an ionic pair with Gd-DTPA. It was surprisingly discovered that in this fashion the liposome with high therapeutic drug load and high remote marker load can be efficiently produced. These dual-loaded liposomes can be used as self-reporting, remotely detectable drugs per se, or in combination with ligand-directed targeting to pathologic cells.

### Liposomes encapsulating remotely detectable probes sensitive to cell internalization

As discussed previously, one aspect of this invention is a liposome for remote sensing of endocytosis comprising a detectable marker whose signal is modulated upon endocytosis of the liposome into a cell of interest. Preferably the detectable marker is paramagnetic metal chelate or a paramagnetic metal chelate conjugated to a lipopolymer, such as gadolinium DTPA-BMA, gadolinium DTPA, or gadolinium HP-DO3A. The liposome may comprise a poorly water-permeable membrane and the detectable marker may be a fluorescent marker. Where the detectable marker is a fluorescent marker, it may be present at a self-quenching concentration or the liposome further comprises a fluorescent quencher. Preferably the liposome comprises pH-sensitive lipopolymers.

Extensive work has been completed in the development of pH-sensitive or reduction-dependent liposomes for enhanced intracellular release of fluorescent probes and therapeutic agents (Chu et al., Pharm. Res. 7, 824-834, 1990; Drummond et al., Biophys. J. 64, A72, 1993; Drummond et al., Biophys. J. 72, A13, 1997; Drummond et al., Progress in Lipid Research 39, 409-60, 2000; Düzgünes et al., Biochemistry 24, 3091-3098, 1985; Düzgünes et al., (1991) in Membrane Fusion (Wilschut, J. and Hoekstra, D., eds.), pp. 713-730, Marcel Dekker, Inc., New York; Kirpotin et al., FEBS Lett. 388, 115-118, 1996; Leroux et al., J. Controlled Rel. 72, 71-84, 2000; Meyer et al., FEBS Lett. 421, 61-64, 1998; Straubinger et al., FEBS Lett. 179, 148-154, 1985; & Zignani et al., Biophys. Acta 1463, 383-394, 2000). These studies provide guidelines concerning the strategies important for intracellular release, while maintaining favorable pharmacokinetic properties *in vivo.* The design of pH-, and thus endocytosis-sensitive liposomes in the past often required extensive modification of the liposome surface, resulting in rapid clearance upon iv administration and thus limiting their potential utility. However, the present invention provides several approaches for making the entire agent pH- or enzyme-sensitive that do not require significant modification of the membrane surface, and thus allow the liposomal carrier to maintain its favorable pharmacokinetic properties. The present invention relies on the enzyme sensitivity of the liposomal membrane itself, resulting in leakage of the contrast agent, i.e. the detectable marker, or simply increasing the permeability to water, or on the enzyme or pH-sensitivity of the quenched agent protected in the interior of the liposome. These approaches allow the probe to have the same pharmacokinetic properties as the therapeutic agent and thus effectively predict the suitability of treatment via this agent.

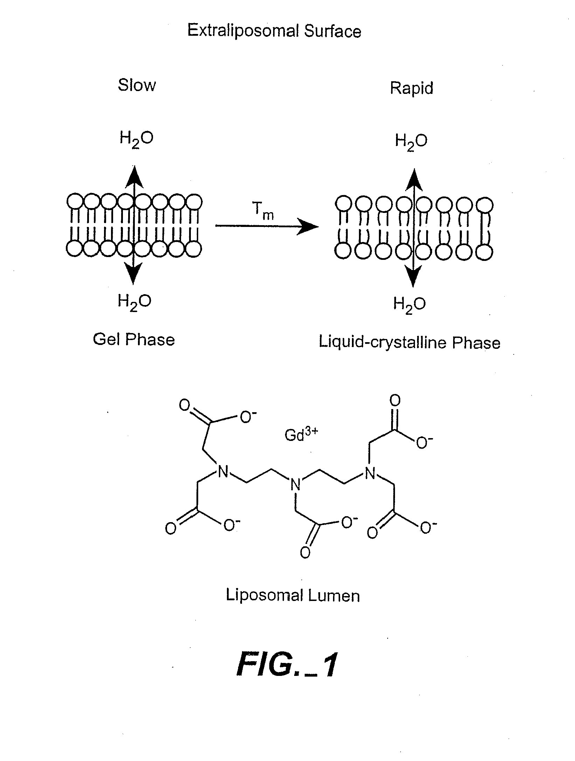

Gd³⁺-containing liposomes composed of cholesterol and high phase transition lipids show a relatively low relaxtivity for Gd³⁺ due to the slow diffusion of water across these membranes. However, liposomes composed of cholesterol and low phase transition phospholipids (those typically in the liquid crystalline phase at 37 °C) show a high relaxivity and a readily detectable signal by MRI. The difference in T1 between the two preparations was approximately 12-fold. Indeed, for simple biodistribution and tumor localization purposes, these liposomes are preferred due to their increased signal strength. However, the effectively quenched signal in the case of high phase transition liposomes offers a unique opportunity to "cage" the detectable signal until the probe is released from the liposomal carrier. See FIG. 1, which depicts liposomes composed of phospholipids that are typically in the gel phase (highly ordered) at 37 °C will have a relatively slow diffusion of water across the liposomal membrane, resulting in a greatly reduced relaxivity, and thus effectively quenching the Gd³⁺ probe. However, liposomes composed of phospholipids that are typically in the liquid crystalline phase (more fluid state) have a significantly greater rate of water exchange across the liposomal membrane and thus an enhanced relaxivity.

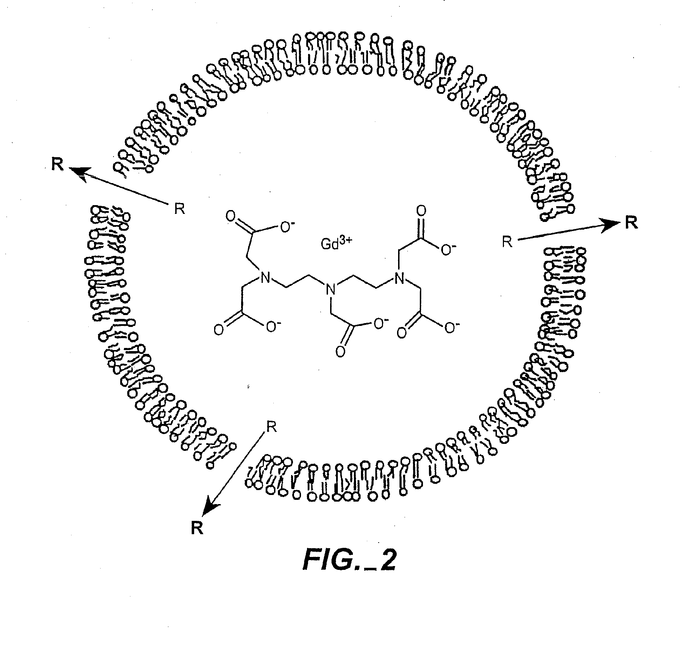

In one embodiment of the invention, this would correspond to recognition of a ligand-targeted liposome by the receptor-overexpressing cancer cell and endocytosis. Upon endocytosis, the lipid membrane will be degraded in the lysosome, effectively "uncaging" the marker, i.e. MRI probe, and resulting in a considerable increase in the detectable signal. In fact, the liposomal carrier need not be degraded to the point where the Gd³⁺-chelates could escape the confines of the carrier, but only to the degree that would allow more rapid exchange of water across the membrane (FIG. 2). Thus, only cells that bind and internalize liposomes will be effectively imaged using this technique.

Many studies have demonstrated degradation of both lipid, and either encapsulated or bound protein following internalization by macrophages, fibroblasts, endothelial cells, and tumor cells (Straubinger et al., 1983; Dijkstra et al., Exp. Cell Res. 150, 161-176, 1984; Jett et al., Cancer Res. 45, 4810-4815, 1985; Storm et al., Biochim. Biophys. Acta 965, 136-145, 1988; Derksen et al., Biochim. Biophys. Acta 971, 127-136, 1988; Trubetskaya et al., FEBS Lett. 228, 131-134, 1988; Chu et al., Pharm. Res. 7, 824-834, 1990). Increased release of doxorubicin from liposomes was observed following uptake by peritoneal macophages, and collected supernatants were shown to have considerable growth-inhibitory activity (Storm et al., Biochim. Biophys. Acta 965, 136-145, 1988). The degradation rate was dependent on the lipid composition of the liposomes, with liposomes containing high phase transition phospholipids (slow release) being degraded more slowly than those containing low phase transition phospholipids (rapid-release; Storm et al., Biochim. Biophys. Acta 965, 136-145, 1988).

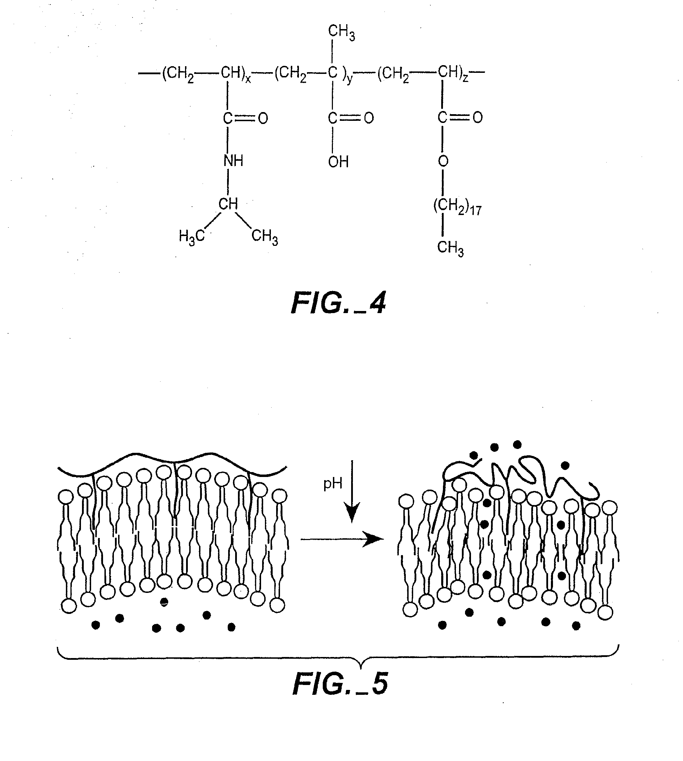

Another embodiment of this invention provides reduced permeability liposomes modified with pH-sensitive copolymers. pH-responsive copolymers have the advantage over many other approaches for creating pH-sensitive liposomes, in that they can be used to prepare liposomes of almost any composition, thus reducing the effect of the modification on pharmacokinetics and contents leakage. The consequence of acid titration is a modification of the polymer conformation and its association with the liposomal membrane, resulting in its destabilization and contents leakage (Figure 5). Copolymers of N-isopropylacrylamide (NIPAM) and methylacrylic acid (MAA) have been used as one example of a pH-sensitive copolymer. These copolymers can be anchored in liposomal membranes using hydrophobic anchors such as octadecylacrylate (ODA). An example of one of these copolymers, poly(NIPAM)co-MAA-co-ODA, is shown in Figure 4. Other examples of pH-sensitive copolymers include succinylated poly(glycidol)s, poly(acrylic acid) derivatives, and poly(histidine).

### Enzymatically Caged Paramagnetic Chelates

In another embodiment of this invention, pH- or enzyme-sensitive shielding groups will be used to "cage" the paramagnetic properties of the chelated ion. Release of the shielding group intracellularly upon exposure to low pH or enzymes located in intracellular organelles, such as the lysosomes or late endosomes, will result in a change in relaxivity for water molecules. Meade and colleagues have recently described a β-galactosidase-sensitive Gd³⁺ chelator that was used to detect expression of the β-galactosidase gene noninvasively using MRI (Moats et al., Angew Chem. Intl. Edn. Engl. , 726-728, 1997; Louie et al., Nature Biotechnology 18, 321-5, 2000). In this compound, a galactopyranose molecule is conjugated and positioned on the chelator to block the final coordination site on Gd³⁺ to water, thus effectively reducing the relaxivity by a factor of up to three. Hydrolysis of the galactopyranose molecule results in the availability of this final coordination site and an increase in the relaxivity. A similar series of compounds that are cleavable by glucuronidase have been synthesized and demonstrate similar effects on relaxivity. Glucuronidase is present in the lysosomes and possibly late endosomes of cells (Dutton, (1966) Glucuronic acid, free and combined, Academic Press, New York; Fishman, (1970) Metabolic conjugation and metabolic hydrolysis, Academic Press, New York) and thus would be an effective trigger to indicate endocytosis has occurred. It was also been shown to be released from tumors; being subsequently located in the tumor interstitium. However, encapsulating the conjugated chelator inside liposomes where it would not have access to extracellularly derived glucuronidase, but only lysosomal enzyme, would amplify the signal upon endocytosis due to the requirement of the lipid barrier being broken down for the enzyme to have access to its substrate.

### pH-Caged Paramagnetic Chelates

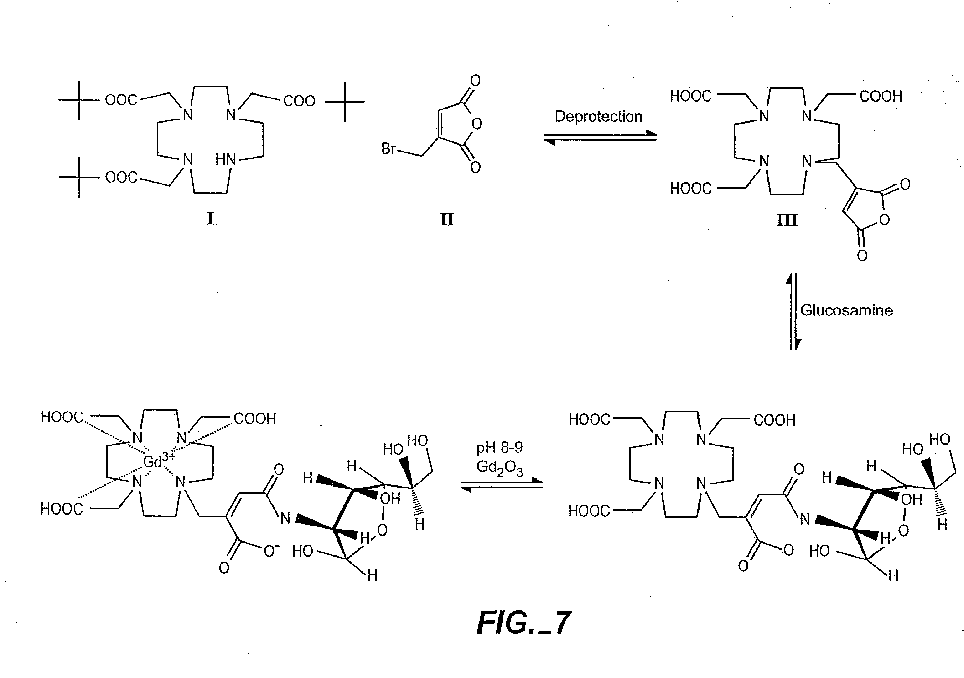

Finally, pH-sensitive probes for non-invasive imaging via positron emission topography (PET), electron paramagnetic resonance (EPR) imaging, and MRI have been described (Helpern et al., Magnetic Resonance in Medicine 5, 302-5, 1987; Morikawa et al., Investigative Radiology 29, 217-23, 1994; Mehta et al., Bioconjugate Chemistry 7, 536-40, 1996; Khramtsov et al., Cellular & Molecular Biology 46, 1361-74, 2000; Mikawa et al., Journal of Biomedical Materials Research 49, 390-5, 2000; Witt et al., Biomaterials 21, 931-8, 2000). Encapsulation of these agents in liposomes semipermeable membrane to hydrogen ions (Biegel et al., Biochemistry 20, 3474-3479, 1981; Clement et al., Biochemistry 20, 1534-1538, 1981) will allow for the noninvasive detection of endocytosis *in vivo.* pH-Sensitive fluorescent probes, including calcein and pyranine, have been used previously in cell culture to observe endocytosis qualitatively by fluorescence microscopy or quantitatively by fluorescence ratio imaging (Daleke et a., Biochim. Biophys. Acta 1024, 352-366, 1990; Straubinger et al., Biochemistry 29, 4929-4939, 1990; Lee et al., Biochemistry 32, 889-899, 1993; Kirpotin et al., Biochemistry 36, 66-75, 1997). However, noninvasive *in vivo* monitoring of endocytosis of liposomes has thus far been an elusive goal. This invention describes a pH-sensitive citraconyl- (Scheme 2, depicted in FIG. 7) or hydrazide or linker to reversibly "cage" Gd³⁺-DTPA chelates in a similar fashion to the enzymatically-sensitive linker described previously.

For the synthesis of the citraconyl-linked conjugate, N-bromosuccinimide was combined with citraconic anhydride in equimolar amounts and then added to a catalytic amount of benzoyl peroxide (initiator) in carbon tetrachloride. The mixture was transferred to a heating mantle and the reaction was refluxed overnight to form the 2-bromocitraconic anhydride **(II).** The purified product was recovered after vacuum distillation and the final yield was 60%. 1,4,7,10-tetracylododecane-1,4,7-tris (acetic acid t-butyl ester) (I, Macrocyclics; Dallas, TX) can be derivatized with the purified 2-bromocitraconic anhydride **(II)** to form the intermediate **III.** The citraconic functionality can then react with glucosamine and gadolinium incorporated to form the pH-sensitive Gd-DOTA contrast agent **(IV).** Under acidic conditions, such as those found in lysosomes or late endosomes, the citraconic linker undergoes an intramolecular reaction resulting in release of the quenching agent (glucosamine) and the free Gd-DOTA chelate.

It has been shown that non-chelating groups of polyaminopolycarboxylic chelates of gadolinium can have substantial effect on the exchangeability of bound water molecules, and that this phenomenon can be used to construct gadolinium probes responsive to pH (Zhang et al. Invest. Radiol. 36:82-86, 2001). Meade's group has shown that polyols (galactose) conjugated to the carboxylic groups of Gd-DOPA complex reduce the relaxivity by blocking water access to the paramagnetic core, and the removal of these polyol groups restores high relaxivity of the Gd-DOTA complex (Louie, et al., Nature Biotechnology 18, 321-5, 2000). Willner, et al. (Bioconj. Chem. 4, 521-527, 1993) has demonstrated bioconjugates that employ a hydrazone bond which is relatively stable at neutral pH and dissociates at pH 5.0-5.5 typical for endosomes and lysosomes. The principle of "pH-caging" of biomolecules has been generally demonstrated (Drummond, 2001). The present invention provides "pH-caged" paramagnetic chelates that create the endocytosis-sensing probes whose relaxivity irreversibly and significantly increases in the acidic environment of endosomes and/or lysosomes. To do so, DTPA mono- and bis-hydrazide is synthesizde by the reaction of DTPA-cyclic anhydride (DTPA-ca) with one or two equivalents of hydrazine in absolute ethanol. Mono- and polysubstituted DOTA hydrazides can be synthesized by the action of hydrazine on NHS-esters of DOTA prepared in the presence of DCC in dioxane/dimethoxyethane. These hydrazide chelators can be complexed to gadolinium in a conventional way. Carbonyl carrying polyol moieties with different carbonyl reactivities can be prepared by the reaction of amino sugar (glusosamine, galactosamine) with NHS esters of ketoacids (levulinic acid, acetoacetic acid, or pyruvic acid) in the presence of triethylamine in DMF. The "caged" conjugates are prepared by forming the hydrazone bond between the ketoacid moiety of the sugar ketoacylamide and the hydrazide group of the hydrazide chelates in alcohol.

Shielding strategies can be sufficient to suppress baseline signal from non-internalized liposomes. However, if only a two- to four-fold increase in signal is achieved using non-shielded liposomes containing pH- or enzyme-sensitive MRI probes, this differential alone may not result in sufficient signal modulation in targeted cells. The strategy that appears particularly promising involves internalization-triggered endosomal disruption of relatively impermeable liposome ("IL") membranes, as described above. Our initial studies suggest at least a 20-fold increase in signal associated with this approach, which provides substantial ability to differentiate internalized vs. non-internalized ILs. This increase is several-fold greater than that reported for other strategies involving target site activated imaging agents. A combined approach can be especially powerful: the liposome shielding approach described can be used in conjunction with encapsulated probes that are in turn intrinsically activatable, resulting in potentially additive or even synergistic gains in signal intensity.

Fluorescent markers are another class of remotely detectable markers suitable for the use in the liposomes according to this invention. Markers which have excitation maxima in the far red or infrared region of the light spectrum are especially suitable due to the deeper penetration of the excitation and emission radiation through the tissues wherein an intravital infrared laser imaging systems known in the art can be used. Carbocyanine dyes such as Indocarbocyanine Green (a fluorescent dye used in blood vessel imaging and cardiovascular diagnostics) are especially suitable. These dyes when entrapped into liposomes at more than 2 mM concentration have surprisingly prominent increase in fluorescence upon release from the liposome. Without being bound by a particular mechanism of action, this phenomenon can be theoretically explained by self-quenching of the liposomally-entrapped dye. Upon endocytosis by the cells of interest, such liposomes are degraded, the dye is released, and the fluorescence increases with time, signifying the presence of such endocytosing cells.

### Radionuclide- or MRI-agent linked liposomes for cell-specific imaging and drug delivery

Capacity of cells to endocytose certain ligands helps to determine pathologic nature of these cells and therefore to improve diagnosis and/or treatment of diseases. According to this invention, endocytosis-sensitive liposomal probes are made specific to certain types of cells, such as cancer cells, by attachment of ligands which are specific to such cells and preferably, also induce a high rate of endocytosis. The kinds of cell-specific ligands and their recognition molecules are well known in the art. In one preferred case, such ligands are antibodies and antibody fragments. Liposomes with attached antibodies or antibody fragments are termed immunoliposomes.

Efficient conjugation methodology is essential for transforming relatively nonreactive liposomes into target-cell internalizable immunoliposomes. Conjugation of targeting moieties, such as, antibodies and antibody fragments, to liposomes can be achieved by any method known or to become known in the art. For example, a micellar incorporation method involving 2-step conjugation of proteins, such as antibody fragments, to preformed drug-loaded liposomes has previously been demonstrated [see U.S. Pat. 6,210,707, which is incorporated herein by reference and figure 2c of Kirpotin et al., 1999]. According to this method, the fragments, such as Fab' or scFv, are first covalently conjugated to terminally derivatized PEG-phosphatidylethanolamine linkers in solution, resulting in micelle-forming immunoconjugates. Next, the conjugates are incorporated into drug-loaded liposomes by co-incubation, resulting in MAb fragments covalently conjugated to the termini of PEG chains and anchored through their lipid moieties to the liposome surface. Anti-HER2 ILs-doxorubicin produced by this method were equivalent in binding, internalization, and *in vivo* efficacy to anti-HER2 ILs-doxorubicin produced by our previous methods, in which MAb fragments were conjugated to specially constructed liposomes containing functional sites (Park et al., J. Control. Release (suppl.), 74: 95-113, 2001). Similar "micellar insertion" methodology is applicable for liposomal attachment of smaller targeting ligands, such as peptides and oligisaccharides (Zalipsky et al., 1998).

The present invention surprisingly demonstrates that chelated imaging agents covalently linked to different lipopolymers, such as, for example, lipids derivatized with polyalkylethers, e.g., poly(ethylene glycol) (pegylated lipids), attach themselves to preformed liposomes or other lipid-based microparticles without the loss of their imaging capacity and without the loss of the liposome encapsulate drug. Co-incubation with such lipopolymer-linked remote sensing probes effectively transforms the targeted or nontargeted liposome or liposomal therapeutic into an imaging agent, capable of reporting on various molecular events, general biodistribution, or therapeutic effectiveness. This is a novel and efficient strategy for transforming liposomal delivery systems into imaging agents. A series of lipopolymer-anchored chelating agents include a variety of lipid anchors and linking groups. For example, a di (C12-C20)alkylamide, such as distearylamide (C18) group, a sterol such as cholesterol, and a di (C12-C20)-acylglycerol derivative, such as distearoylphosphocholine, are suitable lipid anchors; a poly(alkylether), such as poly(ethylene glycol) is a suitable hydrophilic polymer, and NTA, EDTA, DTPA, DO3A or DOTA, as well as their analogs (e.g., phoshonate analogs) and derivatives are suitable chelation groups for radionuclide or paramagnetic metal ions.
As discussed hereinbefore, another aspect of this is invention is liposome comprising (a) a remotely detectable marker and (b) a therapeutic agent. The liposome is configured in accordance with the previous discussion and the detectable marker is a detectable marker whose signal is modulated upon endocytosis of the liposome into a cell of interest and therapeutic agent is an anti-cancer agent. In one embodiment, the therapeutic agent and detectable marker are members of an ionically coupled pair, that is, if, within the liposomal inner space one forms an anion, the other forms a cation, and vice versa. A member of the pair is first encapsulated in the liposome in a form providing for transmembrane ionic, chemical, or electrochemical gradient that enhances the encapsulation of the other member through a highly efficient "remote loading" principle. Optionally, two members form a stable, e.g., precipitated or gel-like, ionic complex, or salt, that also improves the drug encapsulation stability and/or drug efficacy. It was surprisingly discovered that as a member of such ionic pair with a therapeutic agent, e.g., an anticancer drug, the marker, e.g. Gd-polyaminopolycarboxylate paramagnetic chelate, largely retains its detection capacity A variety of anionic paramagnetic chelates are suitable as members of the drug-marker ionic pair. For example, paramagnetic metal complexes of polyaminopolycarboxylic acids (nitrilotroacetate, ethylenediamine tetraacetate, diethylenetriamine pentaacetate), their anionic derivatives and phosphonate analogs are suitable. Typically, a solution of an anionic paramagnetic complex,e.g., a chelate, in the form of a free acid, buffered (e.g., partially neutralized with alkali) acidic solution, or in the form of an ammonium or substituted ammonium salt is first used for entrapment into liposomes. Concentrations of the anionic paramagnetic complex solution are typically at least 0.01 M, but may be at least 0.05 M, 0.1M, or 0.2 M, and the upper limit is dictated by the solubility of the paramagnetic complex, but usually is up to 1.0 M or 2.0 M, but generally no more than 3.0 M. The anionic paramagnetic metal complex is encapsulated into liposomes by any suitable method, e.g. by hydration of the lipids in this solution, followed by size reduction of the particles by mechanic shearing or membrane extrusion. Then the extraliposomal chelate is typically removed, e.g., by dialysis or gel filtration, and the liposomes are incubated with the therapeutic agent in a buffered or unbuffered aqueous solution for a time sufficient for the agent to become co-encapsulated. Typically incubation times from about 5 min. to about 2 hours, and the temperatures of ambient (about 20°C) to about 80°C are suitable to effect co-encapsulation of the agent. While any therapeutic agent that is compatible with the liposome system is useful, it has been found that agents capable of attaining a cationic charge due to electrolytic dissociation, such as doxorubicin, are preferred. Other therapeutic agents suitable for the present invention are, for example, those set forth in "Goodman and Gilman's The Pharmacological Basis of Therapeutics," Tenth Edition, edited by Hardman and Limbird. The anticancer agents at pages 1381-1460 are useful. These pages are incorporated herein by reference. Therapeutic agents that are amphoteric in nature are particularly useful.

Anticancer agents useful in the inention include those listed hereafter.
Structure-based classes: fluoropyrimidines-5-FU, fluorodeoxyuridine, ftorafur, 5'-deoxyfluorouridine, UFT, s-1 capecitabine; pyrimidine nucleosides-deoxycytidine, cytosine arabinoside, 5-azacytosine, gemcitabine, 5-azacytosine-arabinoside; purines-6-mercaptopurine, thioguanine, azathioprine, allopurinol, cladribine, fludarabine, pentostatin, 2-chloro adenosine; platinum analogues-cisplatin, carboplatin, oxaliplatin, tetraplatin, platinum-DACH, ormaplatin, CI-973, JM-216; anthracyclines/anthracenediones and derivatives-epirubicin, doxorubicin, daunorubicin, mitomycin C, pirarubicin, rubidomycin, carcinomycin, n-acetyladriamycin, rubidazone, 5-imidodaunomycin, n-acetyldaunomycine, daunoryline, mitoxanthrone; epipodophyllotoxins-etoposide, teniposide; camptothecin and derivatives-irinotecan, topotecan, lurtotecan, silatecan, 9-amino camptothecin, 10,11-methylenedioxycamptothecin, 9-nitrocamptothecin, TAS 103, 7-(4-methyl-piperazino-methylene)-10,11 -ethylenedioxy-20(S)-camptothecin, 7-(2-N-isopropylamino)ethyl)-20(S)-camptothecin, 7-ethylcamptothecin, 10-hydroxycamptothecin, 9-nitrocamptothecin, 10,11-methylenedioxycamptothecin, 9-amino-10,11-methylenedioxycamptothecin, 9-chloro-10,11-methylenedioxycamptothecin, (7-(4-methylpiperazinomethylene)-10,11-ethylenedioxy-20(S)-camptothecin, 7-(4-methylpiperazinomethylene)-10,11-methylenedioxy-20(S)-camptothecin, 7-(2-n-isopropylamino)ethyl)-(20S)-camptothecin; hormones and hormonal analogues-diethylstilbestrol, tamoxifen, toremefine, tolmudex, thymitaq, flutamide, bicalutamide, finasteride, estradiol, trioxifene, droloxifene, medroxyprogesterone acetate, megesterol acetate, aminoglutethimide, testolactone and others; enzymes, proteins and antibodies-asparaginase, interleukins, interferons, leuprolide, pegaspargase, and others; vinca alkaloids-vincristine, vinblastine, vinorelbine, vindesine; taxanes-paclitaxel, docetaxel.
Mechanism-based classes: antihormonals-see classification for hormones and hormonal analogues, anastrozole; antifolates-methotrexate, aminopterin, trimetrexate, trimethoprim, pyritrexim, pyrimethamine, edatrexate, mdam; antimicrotubule agents-taxanes and vinca alkaloids and derivatives; alkylating agents (classical and non-classical)-nitrogen mustards (mechlorethamine, chlorambucil, melphalan, uracil mustard), oxazaphosphorines (ifosfamide, cyclophosphamide, perfosfamide, trophosphamide), alkylsulfonates (busulfan), nitrosoureas (carmustine, lomustine, streptozocin), thiotepa, dacarbazine and others; antimetabolites-purines, pyrimidines and nucleosides, listed above; antibiotics-anthracyclines/anthracenediones, bleomycin, dactinomycin, mitomycin, plicamycin, pentostatin, streptozocin; topoisomerase inhibitors-camptothecin and derivatives (topo I), epipodophyllotoxins, m-AMSA, ellipticines and derivatives (topo II), ellipticine, 6-3-aminopropyl-ellipticine, 2-diethylaminoethyl-ellipticinium and salts thereof, datelliptium, retelliptine; antivirals-AZT, zalcitabine, gemcitabine, didanosine, and others; miscellaneous cytotoxic agents-hydroxyurea, mitotane, fusion toxins, PZA, bryostatin, retinoids, butyric acid and derivatives, pentosan, fumagillin, and others.
In addition to the above, an anticancer agent includes without any limitation, any topoisomerase inhibitor, vinca alkaloid, e.g., vincristine, vinblastine, vinorelbine, vinflunine, and vinpocetine, microtubule depolymerizing or destabilizing agent, microtubule stabilizing agent, e.g., taxane, aminoalkyl or aminoacyl analog of paclitaxel or docetaxel, e.g., 2'-[3-(N,N-Diethylamino)propionyl]paclitaxel, 7-(N,N-Dimethylglycyl)paclitaxel, and 7-L-alanylpaclitaxel, alkylating agent, receptor-binding agent, tyrosine kinase inhibitor, phosphatase inhibitor, cycline dependent kinase inhibitor, enzyme inhibitor, aurora kinase inhibitor, nucleotide, polynicleotide, and farnesyltransferase inhibitor.
Other therapeutic agents contained in the liposome composition of the present invention include wortmannin, its analogs and derivatives, or pyrazolopyrimidine compounds with the aurora kinase inhibiting properties.

In a broader aspect, a therapeutic agent suitable for the use in the liposome of the present invention includes, without limitation any of the following: antihistamine ethylenediamine derivatives (bromphenifamine, diphenhydramine); Anti-protozoal: quinolones (iodoquinol); amidines (pentamidine); antihelmintics (pyrantel); anti-schistosomal drugs (oxaminiquine); antifungal triazole derivatives (fliconazole, itraconazole, ketoconazole, miconazole); antimicrobial cephalosporins (cefazolin, cefonicid, cefotaxime, ceftazimide, cefuoxime); antimicrobial beta-lactam derivatives (aztreopam, cefmetazole, cefoxitin); antimicrobials of erythromycine group (erythromycin, azithromycin, clarithromycin, oleandomycin); penicillins (benzylpenicillin, phenoxymethylpenicillin, cloxacillin, methicillin, nafcillin, oxacillin, carbenicillin); tetracyclines; other antimicrobial antibiotics, novobiocin, spectinomycin, vancomycin; antimycobacterial drugs: aminosalicyclc acid, capreomycin, ethambutol, isoniazid, pyrazinamide, rifabutin, rifampin, clofazime; antiviral adamantanes: amantadine, rimantadine; quinidine derivatives: chloroquine, hydroxychloroquine, promaquine, qionone; antimicrobial qionolones: ciprofloxacin, enoxacin, lomefloxacin, nalidixic acid, norfloxacin, ofloxacin; sulfonamides; urinary tract antimicrobials: methenamine, nitrofurantoin, trimetoprim; nitroimidazoles: metronidazole; cholinergic quaternary ammonium compounds (ambethinium, neostigmine, physostigmine); anti-Alzheimer aminoacridines (tacrine); anti-Parkinsonal drugs (benztropine, biperiden, procyclidine, trihexylhenidyl); anti-muscarinic agents (atropine, hyoscyamine, scopolamine, propantheline); adrenergic dopamines (albuterol, dobutamine, ephedrine, epinephrine, norepinephrine, isoproterenol, metaproperenol, salmetrol, terbutaline); ergotamine derivatives; myorelaxants or curane series; central action myorelaxants; baclophen, cyclobenzepine, dentrolene; nicotine; beta-adrenoblockers (acebutil, amiodarone); benzodiazepines (ditiazem); antiarrhythmic drugs (diisopyramide, encaidine, local anesthetic series-procaine, procainamide, lidocaine, flecaimide), quinidine; ACE inhibitors: captopril, enelaprilat, fosinoprol, quinapril, ramipril; antilipidemics: fluvastatin, gemfibrosil, HMG-coA inhibitors (pravastatin); hypotensive drugs: clonidine, guanabenz, prazocin, guanethidine, granadril, hydralazine; and non-coronary vasodilators: dipyridamole.

An exemplary embodiment includes a liposome with encapsulated anionic MRI marker, Gd-DTPA, in the form of an ammonium or substituted ammonium, e.g., triethylammonium, salt. Upon removal of extraliposomal marker, transmembrane gradient of ammonium ion is created that affords highly efficient (e.g. >95%) co-loading of a cationic anticancer drug, such as doxorubicin, in the same liposome. The resulting dual-loaded liposome is stable against leakage of both drug and paramagnetic marker in vivo, retains cytotoxic properties of the drug, as demonstrated by targeted killing of antigen-expressing tumor cells by antibody-linked drug-marker co-loaded liposomes, and, according to its magnetic relaxivity, allows non-invasive monitoring of the therapeutic agent in a patient.

### Method of the Invention

Another aspect of the invention is a method for non-invasive monitoring of a liposomal drug in a patient's body comprises: (a) administering a liposome comprising, (i) a remotely detectable marker whose signal is modulated upon endocytosis of the liposome into a cell of interest, wherein said remotely detectable marker is conjugated to a lipopolymer and (ii) a therapeutic agent and (b) detecting the signal of the remotely detectable marker. The liposomes discussed hereinbefore are particularly valuable in this method.

Another aspect of this invention is a method for remote sensing of endocytosis of a liposome. The method comprises (a) contacting a cell with a liposome comprising a lipopolymer conjugated to a detectable marker whose signal is modulated upon endocytosis of the liposome into a cell of interest under conditions in which endocytosis can occur and (b) detecting the signal of the detectable marker after endocytosis. The method works particularly well, wherein the detectable marker is paramagnetic gadolinium chelate comprising gadolinium DTPA-BMA and gadolinium HP-DO3A, and the liposome comprises a poorly water-permeable membrane. The detecting step of the method advantageously employs an increase in proton relaxivity by MRI method. The detectable marker is preferably a fluorescent marker and the liposome further comprises a fluorescent quencher. The increased fluorescence activity is then detected by laser imaging methods. Alternatively the detectable marker is a fluorescent marker and is present at a self-quenching concentration. The increased fluorescence activity is detected by laser imaging methods. The method is particularly useful wherein the liposome comprises a cell-internalizable ligand.

### EXAMPLES

The following examples are given to provide representative compounds included as part of this invention. The examples also provide descriptions of *in vitro* and *in vivo* assays to aid in determining the utility of the compounds.

### EXAMPLE 1

### Preparation of Caged Liposomal Carriers

Multiple formulations of low permeability Gd-liposomes were prepared. Multiple liposome formulations of Gd-chelates have been prepared. These liposomes were composed of distearoylphosphatidylcholine (DSPC), cholesterol, and N-poly(ethylene glycol)-distearoylphosphatidylethanolamine (PEG2000-DSPE) or mixtures of DSPC, dipalmitoylphosphatidylcholine (DPPC), and cholesterol (phospholipid:cholesterol (3:2). DPPC was added at small molar ratios to increase the rate of degradation upon reaching the endosomes. However, DPPC does have a lower phase transition than DSPC, and will likely increase the rate of diffusion of water molecules across the liposomal membrane. It remains to be seen to what extent this modification will have on the change in relaxivity upon encapsulation. This is currently being studied and will be compared to other liposomes of varying lipid composition in attempt to optimize the rate of lipid degradation and water permeability. Finally, control liposomes composed of cholesterol and low phase transition phospholipids, such as egg-derived phosphatidylcholine or dioleoylphosphatidylcholine were prepared.

Methods for their preparation included hydration of preformed liposomes with a commercially available gadolinium chelate, Omniscan (Amersham Health; Princeton, NJ) and extrusion of the resulting lipid suspensions through polycarbonate filters of defined pore sizes (0.05 - 0.1 µm). The size of the resulting liposomes varied from 79-115 nm, depending on the pore size of the filter utilized. Unencapsulated contrast agent was removed by a combination of gel filtration chromatography and dialysis. Anti-HER2 scFv (F5)-PEG-DSPE was prepared as previously described (Kirpotin et al., Biochemistry 36, 66-75, 1997, Nielsen et al., Biochimica et Biophysica Acta 1591:109-118, 2002) and inserted into these liposomes by micellar incorporation. Briefly, the conjugate was mixed with the liposomal contrast agent and subsequently incubated at 60 °C for 30 min to allow for the spontaneous transfer of the conjugate from micelles into the outer monolayer of the liposomal membrane. Liposomes containing the conjugated material were then purified on a Sepharose 4B size exclusion column to remove any uninserted conjugate or free scFv.

An important modification came from the substitution of the Gd-10-(2-hydroxypropyl)-1,4,7,19-tetraazacyclododecane-1,4-7-triacetate (HP-DO3A) (PROHANCE, Bracco Diagnostics) for gadolinium diethylenetriamine pentaacetate bismethylamide (Gd-DTPA-BMA) (OMNISCAN, Amersham Health). The change in ionic character together with the five-fold reduction in calcium content in PROHANCE compared to OMNISCAN allow for preparation of liposomal gadolinium formulations with more than twice the Gd per lipid. More precisely, the phospholipid-to-Gd ratio, as determined by NMR was approximately 4 for OMNISCAN and 1.7-1.8 for PROHANCE.

Liposomal PROHANCE was formed using a DSPC/Chol/PEG-DSPE (3:2:0.015, mol:mol:mol) lipid composition and undiluted PROHANCE (279.3 mg/ml gadoteridol, 0.23 mg/ml calteridol, 1.21 mg/ml tromethylamin, 640 mOsmolar, pH 7.4) and extrusion of the resulting lipid suspensions through polycarbonate filters of defined pore sizes (0.05 - 0.1 µm). The size of the resulting liposomes varied from 79-115 nm, depending on the pore size of the filter utilized. Unencapsulated contrast agent was removed by gel filtration chromatography using Sephadex G-75 eluted with Hepes buffered saline (pH 6.5). Anti-HER2 scFv (F5)-PEG-DSPE was inserted into these liposomes by micellar incorporation at 60 °C for 30 min where necessary. Liposomes containing the conjugated material were then purified on a Sepharose 4B size exclusion column to remove any uninserted conjugate or free scFv.

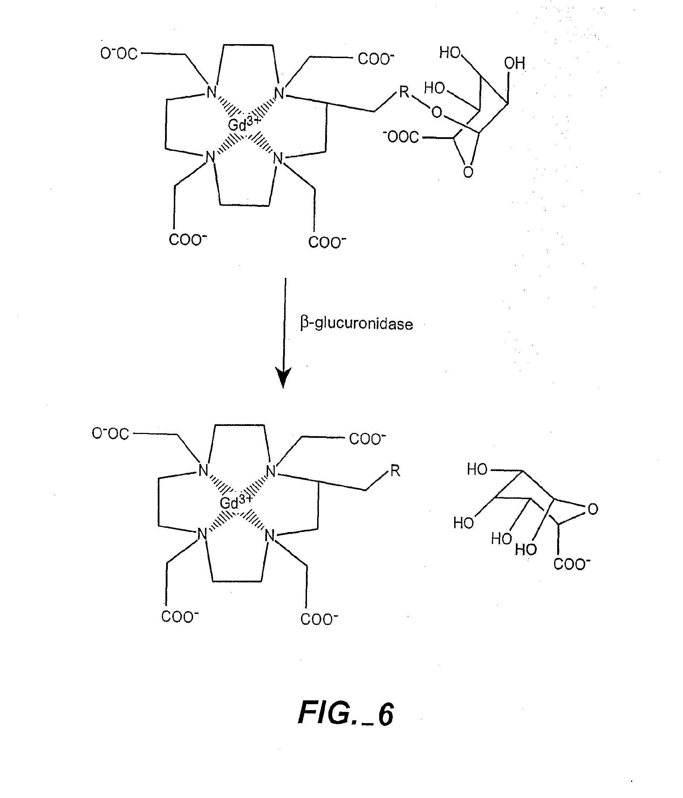

Several pH-sensitive liposome formulations capable of rapidly releasing their contents upon acidification in endosomal or lysosomal lumens were also prepared. These formulations required the incorporation of the pH-sensitive copolymer, poly(NIPAM-co-MAA-co-DODA) (Figure 6) at ratios of 0.05-0.3 (w/w copolymer:total lipid) into the liposomal formulation. The dioctadecylacrylate helped anchor the pH-sensitive copolymer into the lipid membrane. The synthesized DODA-poly(NIPA-co-MAA) had 5 % methylacrylic acid (MAA) and a molecular weight of approximately 15,000.

The copolymer was included either during the liposomal preparation, where it had access to both monolayers of the liposomal membrane, or was inserted into the estraliposomal leaflet after liposome formation by incubation at 60 °C for 30 min. For inclusion during the liposome preparation, the copolymer was dissolved directly in Prohance and the pH of the Prohance solution was kept around 7.4. For insertion following liposome formation, the copolymer was dissolved in Hepes buffered dextrose (5 mM Hepes, 5 % dextrose, pH 7.4). The concept revolves around the idea that at acidic pH, the methylacrylic acid becomes protonated and the copolymer collapses and disrupts membrane integrity (Figure 7), increasing the permeability of the membrane to the liposomal contents or simply to water in general, with the net effect of a rapid change in relaxivity.

### In Vivo Release Characterization

As illustrated in Figure 2 above, a large change in relaxivity upon release from the liposome is expected. Several studies examined what effect endocytic release conditions (low pH initially) had on Gd release from liposomes. Initially, liposomes loaded with quenched fluorescent dyes were used to screen *in vitro* release since the sensitivity and throughput would be considerably greater than if always measuring Gd by NMR. Thus, it is possible to effectively screen a large number of conditions and chose the best for characterization my magnetic resonance. The release of entrapped fluorophores is expected to be similar to that of entrapped imaging agents. In the fluorescence assay, the water soluble pyranince (or HPTS) is coencapsulated with the water soluble quencher, p-xylene-bis-pyridinium bromide (DPX). The degree of quenching is distance dependent, so upon release from the liposome and dilution of the probes there is a large increase in fluorescence.

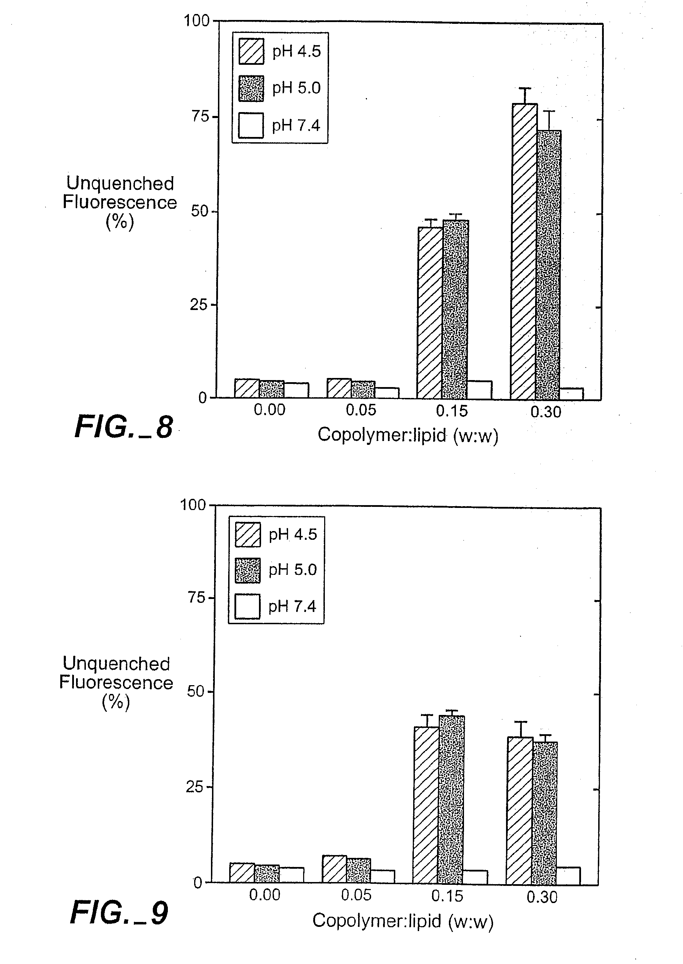

pH can have an effect on the release of HPTS from liposomes that had been modified with the pH-sensitive copolymer at various ratios of copolymer-to-lipid (0.05-3 w/w; Figures 8 and 9). DODA-poly(NIPA-co-MAA). In both figures, it is obvious that unmodified DSPC/Chol liposomes were extremely stable at any pH. Although these incubations are for short times, incubations for as long as 8 h, produced insignificant leakage from these liposomes. However modification of the liposomes with copolymer resulted in rapid and considerable release of HPTS at copolymer-to-lipid ratios of 0.15-0.3 (w/w). The extent of release at pH 4.5 was approximately 73-78 % for liposomes prepared in the presence of the copolymer (Figure 8) and 40-45 % for liposomes prepared where the copolymer was inserted after liposome formation (Figure 9). The release at neutral pH was insignificant in both cases, indicating stability at pH values that would be encountered while traveling in the plasma prior to reaching the tumor and being endocytosed.

The copolymer-modified liposomes were then characterized with respect to their effect on release of Gd from DSPC/Chol/PEG-DSPE liposomes (3:2:0.015). Liposome samples were incubated in this case for 30 min at 37 °C and then characterized by NMR. After initial determination of the relaxivity in the incubated samples, the liposomes were solubilized by addition of Triton-X100 and incubation at 70 °C for 5 min after which the relaxivity was measured again. The results are shown in the table below. It can be readily observed that there is little release during this time for unmodified liposomes. The extent of Gd release or increased water permeability appears to increase for both the liposomes where copolymer was incorporated during extrusion and those where the copolymer was incorporated by insertion when the amount of copolymer incorporated is increased. The degree of change at pH 5.0 was significantly greater for the liposomes with copolymer incorporated during extrusion than those incorporated during insertion. However there does appear to be significant leakage or increased water permeability even at pH 7.4 for the liposomes where the copolymer was incorporated during extrusion. A more comprehensive study looking at stability over longer periods in the presence of human plasma is also studies in hopes of finding a compromise between stability at neutral pH and pH-sensitivity.

| Copolymer-to- lipid (w/w) | pH | ΔR1 intact | ΔR1 solubilized | ΔR1 sol /ΔR1 int |
|---|---|---|---|---|
| 0 | 5.0 | 0.106 | 2.323 | 21.92 |
| | 7.4 | 0.106 | 2.202 | 20.77 |
| | | | | |

| Inserted | | | | |
|---|---|---|---|---|
| 0.05 | 5.0 | 0.134 | 2.466 | 18.40 |
| | 7.4 | 0.110 | 2.563 | 23.30 |
| | | | | |
| 0.1 | 5.0 | 0.121 | 2.261 | 18.69 |
| | 7.4 | 0.102 | 2.241 | 21.97 |
| | | | | |
| 0.15 | 5.0 | 0.163 | 2.302 | 14.12 |
| | 7.4 | 0.099 | 2.409 | 24.33 |
| | | | | |
| 0.2 | 5.0 | 0.318 | 2.241 | 7.05 |
| | 7.4 | 0.086 | 2.202 | 25.6 |
| | | | | |

| During extrusion | | | | |
|---|---|---|---|---|
| 0.05 | 5.0 | 0.535 | 2.344 | 4.38 |
| | 7.4 | 0.219 | 2.398 | 10.95 |
| | | | | |
| 0.1 | 5.0 | 1.791 | 2.990 | 1.67 |
| | 7.4 | 0.352 | 3.162 | 8.98 |
| | | | | |
| 0.15 | 5.0 | 2.586 | 3.455 | 1.34 |
| | 7.4 | 0.433 | 3.498 | 8.08 |
| | | | | |
| 0.2 | 5.0 | 2.429 | 3.126 | 1.29 |
| | 7.4 | 0.422 | 3.135 | 7.43 |

### EXAMPLE 2

### Synthesis of bis-carboxymethyl-PEG600-mono(N,N-dioctadecyl)amide (DSA-PEG-COOH).

3.039 g of bis-carboxymethyl-poly(ethylene glycol) (M.w. 600; Aldrich p/n 40,703-8) were dissolved in 20 mL of chloroform, and stirred with 250 mg of DCC at room temperature for 1 hour. The precipitated urea was filtered out using glass fiber filter, the filtrate was cooled down in an ice bath, mixed with 1 mL of anhydrous triethylamine, and 520 mg of dioctadecylamine (Fluka p/n 42358). The reaction mixture was brought to 40-45°C with stirring unitl all amine was dissolved, overlaid with argon, and stirred at room temperature overnight. The precipitated additional amount of DCU was filtered out, the filtrate was diluted with 20 mL of chloroform and washed consequently with 100 mL of water (3 times), 100 mL of saturated NaHCO₃ (1 time), and 100 mL of water (3 times). Organic layer was dried over sodium sulfate, the solvents were removed on a rotary evaporator, and additionally at 1-2 mm Hg and 50°C for 30 min. Yield of the crude product 886 mg. This product showed two spots (main, Rf=0.34; minor, Rf=0.74) on TLC (Silica, chloroform-methanol 80:20; developed by iodine vapor). This product was dissolved in 5 ml of chloroform and applied on the column containing 20 g of SelectoSilica in chloroform-methanol 80:20 (by volume), and eluted with the same mixture. Ten ml fractions were collected. The product that appeared in fractions 3-6 contained the Rf 0.74 component. Fractions 7-19 that contained chromatographically pure product were combined, reduced to dryness, and dried in vacuum to give the yield of 317.5 mg. This material was dissolved in 3.1 mL of chloroform to give 100 mg/mL solution, passed through 0.2-µm PTFE filter, and stored at -20°C.

### EXAMPLE 3

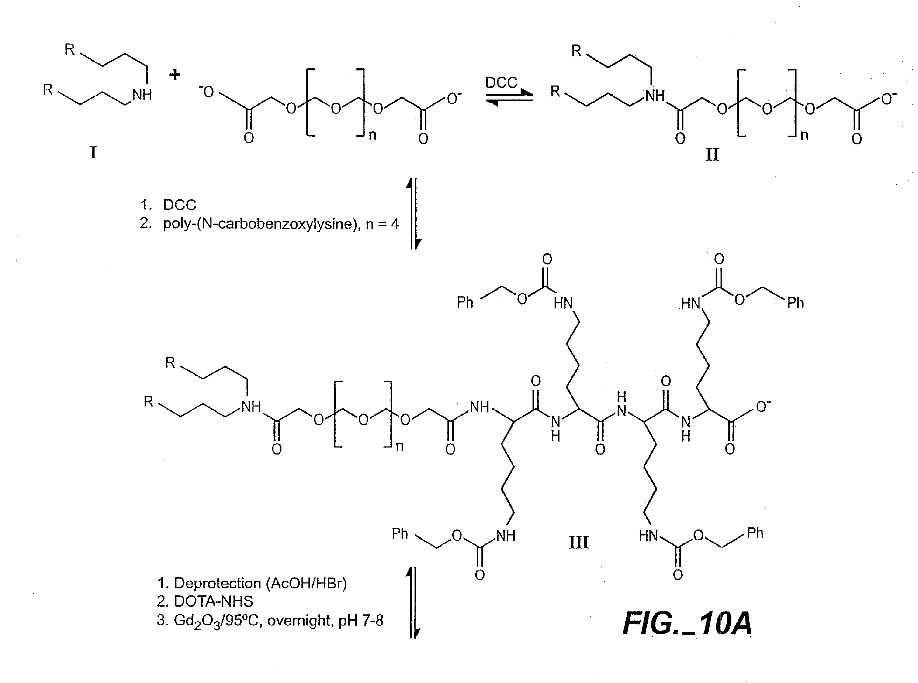

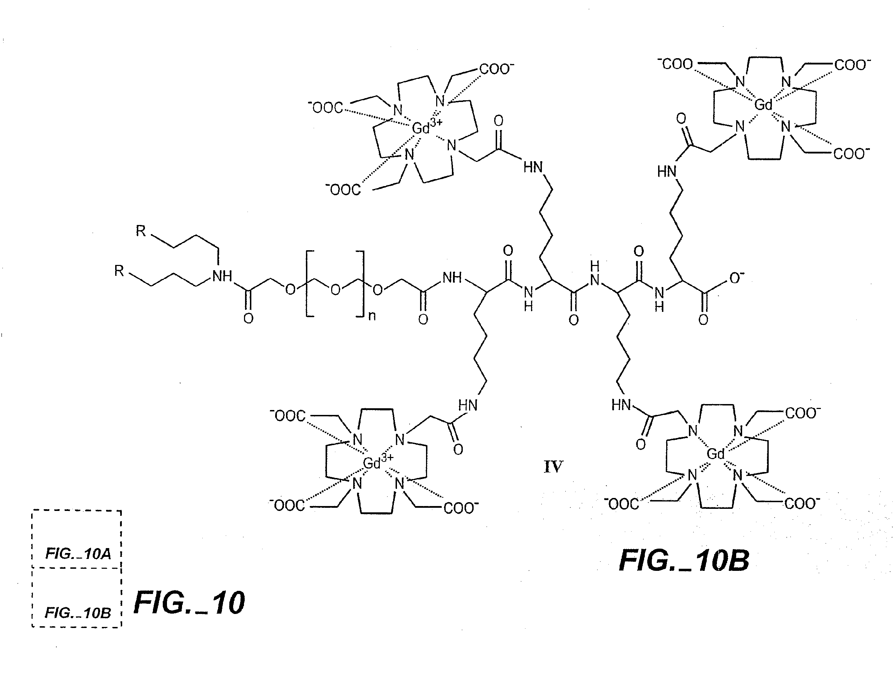

### Synthesis of bis-carboxymethyl-PEG mono-(N,N-dioctadecyl)amide tetra(carbobenzoxy-L-lysine) conjugate (DSA-PEG-(Z-Lys)₄) (Fig. 10)

55 mg of DSA-PEG-COOH in 0.55 mL of chloroform were combined with the solution of 7.9 mg of N-hydroxysuccinimide in 0.5 mL of chloroform. With stirring at room temperature, 13.1 mg of DCC in 0.3 mL chloroform were added dropwise to this solution, and stirred for 2 hours. The precipitate of dicyclohexylurea was separated by filtration through GF/c glass fiber filter. The filtrate was mixed with the solution of 50 mg of poly(N-carbobenzoxy-L-lysine) Mol. weight 1,000 (Sigma) in 0.5 mL of anhydrous dimethylformamide (DMF). The chloroform was removed in vacuum, and additional 1 mL of DMF was added to the residue. 0.042 ml of triethylamine was added to this solution, and stirred overnight under argon. The solvent was removed under vacuum (1-2 mm Hg) at 50°C, and the residue was treated with 4 M HBr in glacial acetic acid for removal of the carbobenzoxy group.

Deprotection of **III** was performed in 4 ml of 30 wt % HBr in acetic acid (4 M) (Aldrich) by stirring at room temperature for 8 h. The solvent was removed under vacuum followed by the addition of 4 ml water, which was titrated to pH 7 with 1 M NaOH. The material was dissolved upon heating to 45 °C for 15 min and clarified by centrifugation (10,000 RPM for 5 min). The supernatant was chromatographed on a 115 ml Sephadex G75 column and collected in 4 ml fractions. The sample containing fractions, as determined by scattering intensity measured spectrophotometrically at 250 nm, were combined and the solvent removed under vacuum to yield 71.6 mg of the deprotected material.

1,4,7,10 tetraazacyclododecane-1,4,7-tris(acetic acid-t-butyl ester)-10-acetic acid mono (N-hydroxysuccinimidyl ester) was obtained from Macrocyclics (Dallas, TX). 119 mg of DOTA-NHS (238 µmol, 50% excess) and 2.14 mmol of triethylamine were added to 71.6 mg of *N*-poly(lysine)-poly(ethylene glycol)-dioctadecylamine in anhydrous DMF. The reaction mixture was heated to 45 °C for 6 h. After removal of the DMF by rotary evaporation, **IV** was dissolved in 4 ml of water by titration to pH 7 with NaOH followed by heating to 45 °C for 15 min. The solution was clarified by centrifugation (10,000 RPM for 5 min). The supernatant was chromatographed on a 115 ml Sephadex G75 column and collected in 4 ml fractions. The sample-containing fractions were determined by scattering intensity measured spectrophotometrically at 250 nm and TLC. Solvent removal yielded 152 mg of light yellow flake material. Product purity was confirmed using TLC. Chromatographic conditions: C-18 reverse phase silica with a EtOH/H₂O (4:1) mobile phase, Rf=0.31, EtOH/H₂O (1:1) mobile phase, Rf=0.91. The product gave a negative response to fluorescamine dye indicating complete 1° amine functionalization.

### EXAMPLE 4

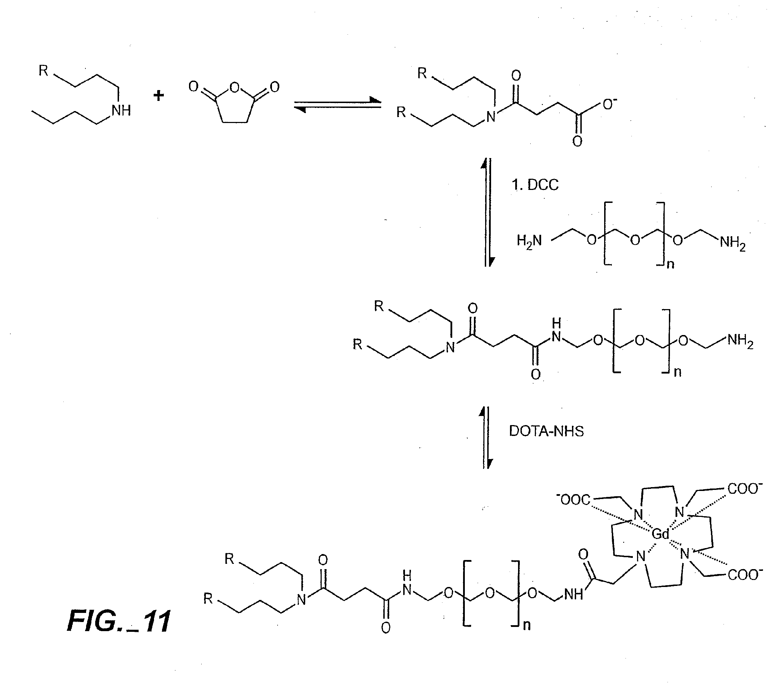

### Synthesis of N-DOTA-poly(ethylene glycol)-dioctadecylamine (Fig.11)

1.6 g of finely ground succinic anhydride was dissolve in 20 mL of anhydrous pyridine. 2.62 g of dioctadecylamine was added, overlaid with argon, warmed up to 40-45°C until the amine dissolved, and stirred at room temperature overnight. The pyridine was removed in vacuum (1-2 mm Hg) at 50°C using rotary evaporator, and the dry residue was dissolved in 50 mL methylene dichloride. The solution was washed 3 times with 100 mL of 1 M aqueous triethylamine bicarbonate, dried over anhydrous sodium sulfate, the solvent was removed on a rotary evaporator, and the solid material was dried overnight in vacuum (0.4 mm Hg) at room temperature. The product showed one spot on TLC (Silica, chloroform-methanol 90:10), Rf=0.82; N,N-dioctadecylamine in the same system, Rf=0.64. Yield 2.709 g (87% of theory). Analytical data: ¹H-NMR (CDCl₃, 400 MHz) δ0.88 (t, 6H, CH₂-C*H*₃), 1.26 (s, 60H, CH₂), 1.56 (q, 4H, OC-N(CH₂-C*H*₂-CH₂)₂), 2.69 (s, 4H, CO-C*H*₂-C*H*₂-CO), 3.23 (t, 2H, OC-N(C*H*₂-CH₂-CH₂)₂), 3.32 (t, 2H, OC-N(C*H*₂'-CH₂-CH₂)₂), 11.9 (br, 1H, COO*H*).

36.5 mg (177 µmol, 10% excess) dicyclohexylcarbodiimide (99% Aldrich, Milwaukee, WI) was added to 100 mg dioctadecyl succinate (161 µmol) and 20.3 mg (177 µmol, 10% excess) *N*-hydroxysuccinimide (97% Aldrich, Milwaukee, WI) dissolved in anhydrous CHCl₃/ethylene glycol dimethyl ether (3:1). After stirring at room temperature for 12 h the mixture formed a colorless solution containing a white precipitate (dicyclohexylurea (DCU)). The reaction mixture was filtered directly into an anhydrous CHCl₃/ethylene glycol dimethyl ether (3:1) solution containing 368 mg (242 µmol, 50% excess) bis(3-aminopropyl) terminated poly(ethylene glycol) (Aldrich, Milwaukee, WI). After stirring for 12 h the solvent was removed by rotary evaporation. The resulting material was dissolved in 3 ml of water and chromatographed on a 100 ml Sephadex G75 column and collected in 4 ml fractions. The sample containing fractions, as determined by TLC, were combined and the solvent removed under vacuum to yield 82.5 mg of white flake product (aminopoly(ethylene glycol)dioctadecylamine). 23.9 mg of 1,4,7,10 tetraazacyclododecane-1,4,7-tris(acetic acid-t-butyl ester)-10-acetic acid mono (N-hydroxysuccinimidyl ester) (47.6 µmol, 20% excess) and 443 µmol of triethylamine were added to 82.5 mg of aminopoly(ethylene glycol)dioctadecylamine in anhydrous DMF. After stirring for 12 h at room temperature the solvent was removed under vacuum yielding a light brown cake. 4 ml of water was added to the solid cake, titrated to pH 7 with NaOH and heated at 45 °C for 10 min. The solution was clarified by centrifugation (10,000 RPM for 5 min) and the light yellow supernatant was chromatographed on a 100 ml Sephadex G75 column and collected in 4 ml fractions. TLC was used to determine the product containing fractions, which were combined and the solvent removed under vacuum yielding 29.9 mg of white flake material. Product purity was confirmed using TLC. Chromatographic conditions: C-18 reverse phase silica with a CHCl₃/MeOH (4:1) mobile phase, Rf=0.91. The product gave a negative response to fluorescamine dye indicating complete 1 ° amine functionalization.

### EXAMPLE 5

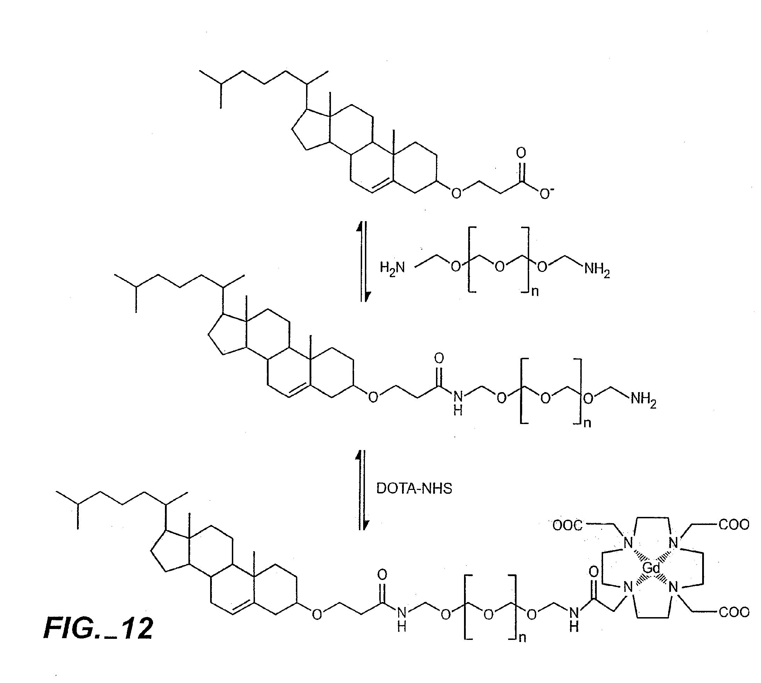

### Synthesis of cholesterol hemisuccinatyl-poly(ethylene) glycol (Fig. 12)

100 mg (205 µmol) of cholesterol hemisuccinate (CHEMS) (Sigma, St. Louis, MO) was dissolved in CHCl₃/ethylene glycol dimethyl ether (3:1) to which was added 26 mg (226 µmol, 10% excess) *N*-hydroxysuccinimide (97% Aldrich) (NHS) and 46.5 mg (226 µmol, 10% excess) dicyclohexylcarbodiimide (99% Aldrich) (DCC) and allowed to stir at room temperature for 6 h resulting in a colorless solution containing a white precipitate. The reaction mixture was filtered directly into a CHCl₃/ethylene glycol dimethyl ether (3:1) solution containing 468 mg (307 µmol, 50% excess) of bis(3-aminopropyl) terminated poly(ethylene glycol) (Aldrich) and allowed to stir at room temperature for 12 h. The solvent was removed by rotary evaporation yielding a light yellow cake. The product was confirmed using TLC without further purification. Chormatographic conditions: using normal-phase silica and a mobile phase consisting of CHCl3/MeOH (4:1) the product Rf=0.59. The amino-poly(ethylene glycol)-cholesterol can be modified with 1,4,7,10 tetraazacyclododecane-1,4,7-tris(acetic acid-t-butyl ester)-10-acetic acid mono (N-hydroxysuccinimidyl ester) to give the DOTA-PEG-Chol derivative. Gadolinium can then be inserted into the chelate by incubating the conjugate with gadolinium oxide at 95 °C overnight at pH 7-8. Free gadolinium is then removed on a Sephadex G-75 column.

### EXAMPLE 6

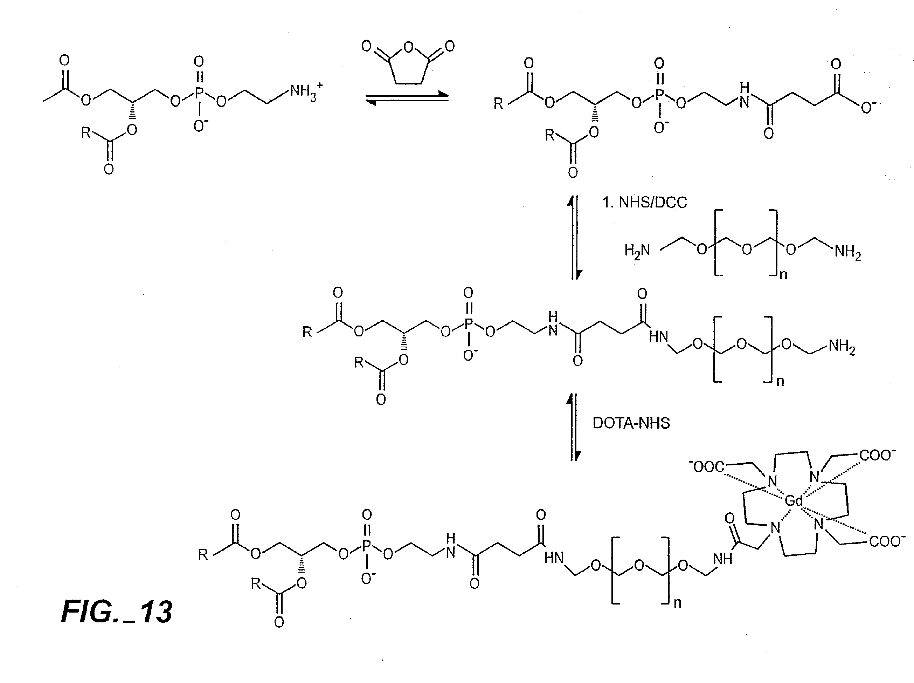

### Synthesis of 1,2-distearoyl-sn-glycero-3-phosphoethanol-N-aminoglutaric acid (Fig. 13)

1,2-distearoyl-sn-glycero-3-phosphoethanolamine (DSPE) was purchased from Sygena. Glutaric anhydride (97%) and triethylamine (99.5%) were purchased from Aldrich. All reagents were used without further purification. A three-fold molar excess of glutaric anhydride (465 mg, 4.08 mmol) and four-fold molar excess of triethylamine (5.44 mmol) were added to DSPE (1.02 g, 1.36 mmol) in anhydrous chloroform and the resulting mixture stirred under argon at room temperature for 12 h. The reaction mixture was washed with 0.1% acetic acid (2) water (2) and pH 9 NaHCO₃ the CHCl₃ fraction was chromatographed on a silica column using hexanes/ethyl acetate (2: 1). The solvent was removed under vacuum to yield a white cake (460 mg, 39.3% theoretical). Product purity was confirmed by TLC and ¹H-NMR. The product gave a negative response to fluorescamine dye indicating complete 1° amine funtionalization. Analytical data: TLC mobile phase hexanes/ethyl acetate (2:1) Rf=0.45, ¹H-NMR (CDCl₃, 400 MHz) δ0.883 (t, 3H, CH₂-C*H*₃), 1.26 (s, 56H, CH₂), 1.61 (br, 4H, O-OC-CH₂-C*H*₂), 1.95 (q, 2H, CO-CH₂-C*H*₂-CH₂-CO), 2.30 (m, 4H, CO-C*H*₂-CH₂-C*H*₂-CO, 4H, O-OC-C*H*₂-CH₂), 3.37 (q, 4H, P-O-C*H*₂-COH₂), 4.16 (q, 1H, CO-O-C*H*₂-CHOCH₂), 4.38 (q, 1H, CO-O-C*H*₂'-CHOCH₂), 5.23 (br, 1H, CO-O-CH₂-CHOCH₂).

The Glu-DSPE derivative can be activated with NHS/DCC and modified with bis(3-aminopropyl) terminated poly(ethylene glycol) (Aldrich, Milwaukee, WI). The amino-poly(ethylene glycol)-DSPE can be modified with 1,4,7,10 tetraazacyclododecane-1,4,7-tris(acetic acid-t-butyl ester)-10-acetic acid mono (N-hydroxysuccinimidyl ester) to give the DOTA-PEG-DSPE derivative. Gadolinium can then be inserted into the chelate by incubating the conjugate with gadolinium oxide at 95 °C overnight at pH 7-8. Free gadolinium is then removed on a Sephadex G-75 column.

### EXAMPLE 7

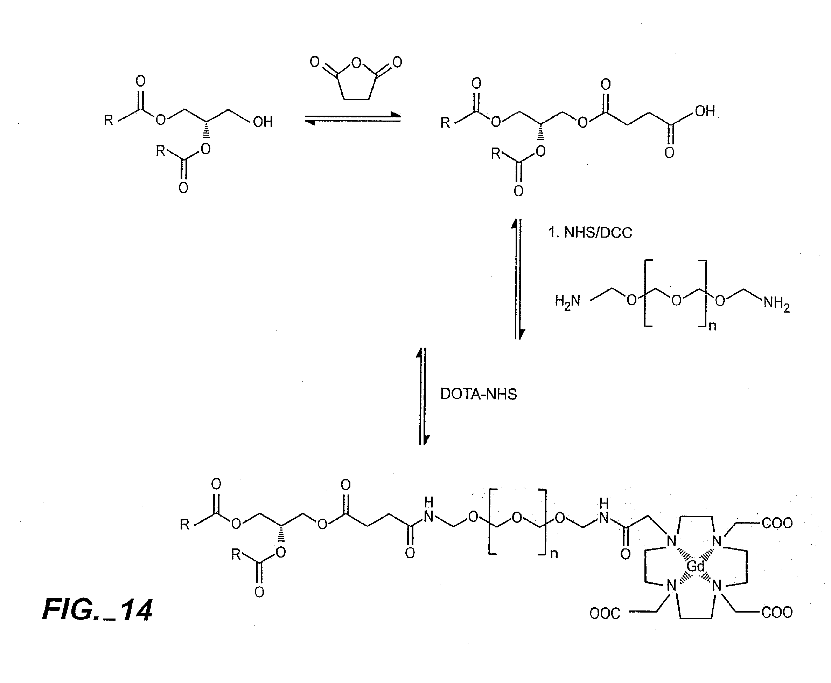

### Synthesis of O-glutaryl-dioctadecylglycerol (glutaryl-DOG) (Fig. 14)

1,2*-O-*dioctadecyl-*sn*-glycerol (99+%) was purchased from BACHEM (King of Prussia, PA). Glutaric anhydride (97%) and 4-dimethylaminopyridine (99+%) were purchased from Aldrich (Milwaukee, WI). All reagents were used without further purification. A two fold molar excess of glutaric anhydride (76.4 mg, 0.76 mmol) and 4-dimethylaminopyridine (81.8 mg, 0.76 mmol) were added to 1,2*-O-*dioctadecyl-*sn*-glycerol (0.2 g, 0.335 mmol) in anhydrous chloroform and the resulting mixture stirred under argon at room temperature for 12 h. The solvent was removed by rotary evaporation and the product purified on a silica column by elution with hexanes/ethyl acetate (2: 1). The product-containing fractions were combined and the solvent removed by rotary evaporation yielding 115 mg (0.162 mmol, 48.4% theoretical) of *O*-glutaryl-dioctadecylglycerol. Product purity was confirmed by TLC and ¹H-NMR. Analytical data: TLC mobile phase hexanes/ethyl acetate (2:1) Rf=0.45, ¹H-NMR (CDCl₃, 400 MHz) δ0.866 (t, 3H, CH₂-C*H*₃), 1.26 (s, 60H, CH₂), 1.55 (q, 4H, O-CH2-CH₂-CH₂), 1.95 (q, 2H, CO-CH₂-C*H*₂-CH₂-CO), 2.40 (s, 4H, CO-C*H*₂-CH₂-C*H*₂-CO), 3.47 (q, 4H, O-C*H*₂-CH₂), 3.57 (t, 2H, CHO-C*H*₂-O), 3.64 (q, 1H, O-C*H*(CH₂)-O), 4.10 (q, 1H, CO-O-C*H*₂-CHOCH₂), 4.23 (q, 1H, COO-C*H*₂'-CHOCH₂), 7.39 (s, 1H, COO*H*).

The Glu-DOG derivative can be activated with NHS/DCC and modified with bis(3-aminopropyl) terminated poly(ethylene glycol) (Aldrich, Milwaukee, WI). The amino-poly(ethylene glycol)-DOG can be modified with 1,4,7,10 tetraazacyclododecane-1,4,7-tris(acetic acid-t-butyl ester)-10-acetic acid mono (N-hydroxysuccinimidyl ester) to give the DOTA-PEG-DOG derivative. Gadolinium can then be inserted into the chelate by incubating the conjugate with gadolinium oxide at 95 °C overnight at pH 7-8. Free gadolinium is then removed on a Sephadex G-75 column.

### EXAMPLE 8

### Therapeutic liposomes with imaging capability: co-encapsulation of imaging and therapeutic agents into immunoliposomes

Liposomes were formed by ethanol injection, followed by repeated extrusion through 0.1 µm polycarbonate filters. Briefly, the dried lipids (DSPC/Chol/PEG-DSPE, 3:2:0.015, mol:mol:mol) were dissolved in 0.5 ml of ethanol by heating at 60 °C. Diethylenetriamine pentacetic acid, gadolinium(III) (Gd-DTPA) and ammonium iron(III) citrate were purchased from Aldrich (Milwaukee, WI). The di-ammonium salt of Gd-DTPA was formed by titration of Gd-DTPA aqueous solution with ammonium hydroxide. Liposomes were hydrated in the presence of 500 mM diammoniumdi-Gd-DTPA or 250 mM ammonium iron (III) citrate and loaded using an ion gradient remote loading method known in the art (Lasic et al., 1992; Haran et al., 1993). However, to our knowledge, this is the first example of using a chelating agent in the dual role of Gd(III) or Fe(III) chelator and drug trapping agent and the use of this method allows for the efficient encapsulation of both the drug and the imaging agent at concentrations that would be necessary for *in vivo* therapy and its simultaneous monitoring.

The 500 mM ammonium-Gd-DTPA or 250 mM ammonium iron (III) citrate solutions (4.5 ml) were heated to 60 °C and then rapidly injected into the ethanolic lipid solutions (0.5 ml, 300 µmol PL) to form a hydrated lipid solution. Ethanol was then removed by rotary evaporation and the lipid suspension was extruded fifteen times through two stacked polycarbonate filters (0.1 µm). Following extrusion, unencapsulated ammonium-Gd-DTPA or ammonium iron (III) citrate was removed on a Sephadex G-75 column eluted with Hepes-buffered saline (pH 6.5). The phospholipid (PL) content was then determined by phosphate analysis (Bartlett, 1959) and doxorubin was added at a ratio of 150 µg drug/µmol PL. The drug was loaded by incubating the drug with the liposomes at 60 °C for 30 min and then subsequently quenching the reaction on ice for 15 min. Unencapsulated drug was removed on a second Sephadex G-75 gel filtration column, also eluted with Hepes-buffered saline, pH 6.5. The amount of drug in the purified liposome samples was quantitated by fluorescence due to intereference of the iron (III) in absorbance measurements (λex = 490 nm, λem=550 nm) following dissolution of the drug-loaded liposomes in acid isopropanol (90 % isopropanol, 10 % 1 N HCl) and phospholipid content was determined by standard phosphate analysis (Bartlett, 1959). Drug loading efficiencies were calculated both in absolute amounts where they are expressed as µg of drug/µmol of phospholipid and in relative terms, where they are expressed as the % of drug loaded relative to the initial amount of drug added. The loading efficiencies for doxorubicin were in the range of 92-100 % when 150 µg drug per µmol phospholipid was initially added. Liposome size was determined by photon correlation spectroscopy, and was in the range of 105-120 nm. The following doxorubicin encapsulation efficiencies were obtained:

| Gradient-forming probe solution | Doxorubicin-to-PL (µg/µmol) | Loading efficiency (%) |
|---|---|---|
| ammonium-Gd-DTPA | 152.9 ± 8.5 | 101.9 ± 5.7 |
| (NH₄⁺)Fe(III)citrate | 138.8 ± 9.9 | 92.6 ± 6.6 |

These liposomes were subsequently characterized by NMR to determine the changes in relaxivity upon solubilization, or complete release of both drug and imaging agent. It was hypothesized that if the magnetic Gd or Fe(III) were caught up in a drug precipitate, that this can affect the relaxivity to an even greater extent than seen due to the effect of reduced water permeability in DSPC/Chol liposomes. However, as can be seen in the table below, the ratio between τ₁ relaxiviy (ΔR1) of the intact and solubilized liposomes, ΔR1sol /ΔR1int ,was similar to that observed previously for non-drug loaded liposomes (Ls-Gd-HP-DO3A). Thus, the liposomes combined the remote endocytosis-sensing properties with high encapsulation of an anticancer drug.

| Gradient-forming probe solution | ΔR1 intact | ΔR1 solubilized | ΔR1 sol /ΔR1 int |
|---|---|---|---|
| NH₄-Gd-DTPA | 0.265 | 6.290 | 23.7 |
| (NH₄)Fe(III)citrate | 0.082 | 0.900 | 13.6 |

### EXAMPLE 9

### Improved MR detectability of GdDTPA-BMA encapsulating Anti-HER2 Immunoliposomes and determination of contribution of antibody targeting to change in tumor MR signal intensity

als of this work were to modify anti-HER2 immunoliposome composition to achieve greater increase in MR tumor signal intensity (SI),evaluate the contribution of anti-HER2 targeting to increase in tumor signal intensity, and determine the relationship between change in signal intensity and measured T1. A greater increase in tumor SI was observed with the new liposomal phospholipid composition compared to previous formulations, however a decrease in liposome diameter did not affect tumor SI significantly. Addition of targeting resulted in an increase in tumor SI compared with non-targeted liposomes. Signal intensity was found to correlate well with measured T1.

Tumor-targeted therapies, such as herceptin and anti-HER2 immunoliposomes, have the potential to play an important role in the selective treatment of cancer. The ability to non-invasively evaluate the distribution of targeted agents can aid in the development and assessment of new targeted therapeutics. The current work explored strategies to improve the MR detectabilty/visibility of 'permeable' ILs in tumors and utilizes 'impermeable' liposomes to assess the contribution of antibody-targeting to the increase in tumor SI seen post-liposome injection. In this study a new composition of permeable (P) GdDTPA-BMA encapsulating anti-HER2 immunoliposomes (ILs), of diameters (80 nm and 50 nm) was evaluated and compared with both targeted (IL) and non-targeted (NTL) water-impermeable (IMP) liposomes in a mouse model of human breast cancer

Four types of GdDTPA-BMA encapsulating liposomes were synthesized: 'permeable' DOPC/Chol/PEG-DPSE (3:1:1) anti-HER2 targeted immunoliposomes (ILs) of two sizes (mean diameter 80 and 50 nm) and 'impermeable' DSPC/DPPC/Chol/PEG-DPSE (2.2:0.3:2:0.3) liposomes (ILs and non-targeted, mean diameter ~75 nm). ILs were targed via and F5 scFv antibody. Nude mice were implanted with the HER2/neu over-expressing human breast cancer line BT474. Tumors were volume matched and mice were imaged in pairs prior to and up to 156 h post-i.v. injection with one of the Gd-liposome compositions (0.05 mmol GdDTPA-BMA/kg). During imaging, mice were anesthetized with 1.5% isoflurane. Imaging was performed on a 1.5T GE Signa scanner (General Electric Medical Systems, Milwaukee, WI) using a conventional wrist coil and customized animal holder. A high resolution coronal 3DFGRE image was acquired at each time point (TR/TE17/4.2ms, FOV 8 cm, matrix 512x256, 4 NEX), T1 was also measured using a 3D variable flip angle fast gradient echo technique. Signal intensity was calculated for regions of interest (ROIS) spanning the whole tumor volume and in blood. T1 was also calculated for tumor ROIs. MR tumor volume was determined by delineating tumor on consecutive slices of a 3D image.

Results: The substitution of DOPC for the POPC resulted in marked increase in average tumor SI change at 24h of +31% (n=5) as compared to the +8% increase seen at 24h with the POPC formulation. Decreasing the liposome diameter from 80 to 50 nm did not result in a significant difference in tumor SI at 24h (31% vs. 28%). Both IMP-ILs and and IMP-NTLs (n=4) resulted similar increases in blood SI at 5 min post-injection (avg 115%) as compared to 'permeable' IL's (245%). The difference between the mean change in tumor SI at 24 h for IMP-ILs (+17%) as compared to IMP-NTLs (+12%) approached significance (p=0.05).

Discussion: The improved increase was found in tumor signal intensity seen for the liposome composition having DOPC layers which is more water-permeable than POPC. The fact that 50 nm P-ILs and 80 nm P-ILs were equally effective for tumor imaging may indicate that 70-80 nm is an optimal size for imaging, compromising between the Gd encapsulation, relaxation and biodistribution properties of the liposomes. The signal intensity change from P-ILs was proportionally higher in both blood and tumors than the IMP-ILs as would be expected due to the decreased permeability of the DSPC phospholipids bilayer to water relative to DOPC. Surprisingly, although the change in blood SI was similar for both IMP-ILs and IMP-Nulls, the IMP-IL's resulted in larger SI increase in tumor, which may reflect the release of the marker triggered by the endocytosis of the ILs into the cell.

### EXAMPLE 10

### Co-loading of doxorubicin and paramagnetic markers Gd-DTPA-(bis)triethylammonium or ferric ammonium citrate into liposomes, and characterization of the resulting drug-marker co-loaded liposomes.

Liposomes were formed by ethanol injection, followed by repeated extrusion through 0.1 µm polycarbonate filters. Briefly, the dried lipids (DSPC/Chol/PEG-DSPE, molar ratio 3:2:0.015) were dissolved in 0.5 ml of ethanol by heating at 60 °C. The triethylammonium salt of Gd-DTPA (TEA-Gd-DTPA) was formed by titration of aqueous solution of Gd-DTPA diacid (Alrdich Corp., Milwakee, USA) with neat triethylamine to pH 5.0-5.4. Liposomes were hydrated in the presence of 500 mM TEA-Gd-DTPA

The 500 mM TEA-Gd-DTPA (4.5 ml) were heated to 60 °C and then rapidly injected into the ethanolic lipid solutions (0.5 ml, 300 µmol PL) to form a hydrated lipid solution. Ethanol was then removed by rotary evaporation and the lipid suspension was extruded fifteen times through two stacked polycarbonate filters (0.1 µm). Following extrusion, unencapsulated TEA-Gd-DTPA or ammonium iron (III) citrate was removed on a Sephadex G-75 column eluted with HEPES-buffered saline (pH 6.5). The phospholipid (PL) content was then determined by spectrophotometric analysis of phosphate following acid digestion (Bartlett GR. 1959. J. Biol. Chem. 234: 466-468) and doxorubicin hydrochloride was added to the liposomes at a ratio of 150 µg drug/µmol PL. The drug was loaded by incubating the drug with the liposomes at 60 °C for 30 min and then subsequently quenching the reaction on ice for 15 min. Unencapsulated drug was removed on a second Sephadex G-75 gel filtration column, also eluted with Hepes-buffered saline, pH 6.5. The amount of drug in the purified liposome samples was quantitated by fluorescence (λex = 490 nm, λem=550 nm) following dissolution of the drug-loaded liposomes in acid isopropanol (90 % isopropanol, 10 % 1 N HCl) and phospholipid content was determined by according to Bartlett (1959). Drug loading efficiencies were calculated both in absolute amounts where they are expressed as µg of drug/µmol of phospholipid and in relative terms, where they are expressed as the % of drug loaded relative to the initial amount of drug added. The loading efficiencies for doxorubicin were in the range of 92-100 % when 150 µg drug per µmol phospholipid was initially added. Liposome size was determined by photon correlation spectroscopy, and was in the range of 105-120 nm.

The liposomes constructed for the previous period that contained either Gd-DTPA/doxorubicin were monitored for stability during storage. After 3 months, the Gd-DTPA/doxorubicin liposomes were similar in size as to when they were prepared (123.9 ± 23.8 vs 121.0 ± 20.7 nm).

Gd-DPTA/doxorubicin were also characterized in an *in vitro* cytotoxicity assay using HER2-overexpressing SKBR3 cells. The results are shown in Figure 16. HER2-targeted immunoliposomes encapsulating Gd-DTPA/doxorubicin were nearly as active as the free drug and one hundred times more active than the nontargeted liposomal formulation. This activity is identical to that seen with conventional HER2-targeted ammonium sulfate-loaded doxorubicin liposomes and demonstrates that the liposomes containing Gd³⁺ maintained both their activity and stability

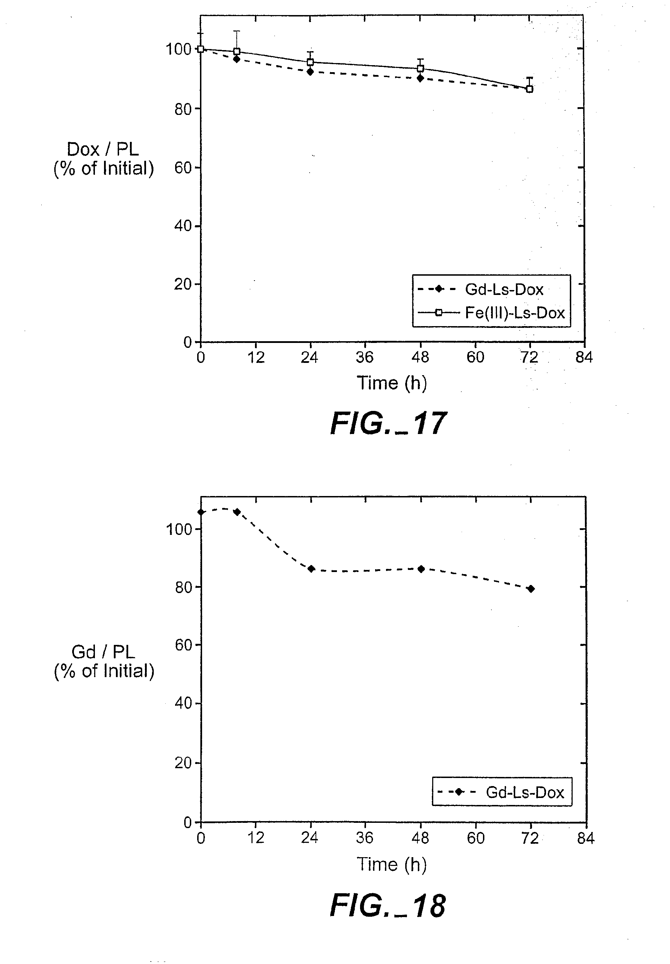

Plasma stability studies were completed in the presence of 50 % human plasma at 37°C using a microdialysis assay. The liposome samples in 50% plasma were placed into sample wells (0.4 ml each) of Spectra/Por Microdialyzer (Spectrum Labs; Gardena, CA) separated from the 32-mL main chamber with 30 nm polycarbonate membrane. The chamber was aseptically charged with 50% (v/v) of human blood plasma(diluted with HEPES-buffered normal saline). Plasma proteins, free (non-encapsulated) drug, and free imaging agent were free to pass through the membrane and accumulate in the lower chamber. The dialyzer was incubated with agitation at 37°C. At prescribed time points, an aliquot of sample from each well was removed and purified by size exclusion chromatography to separate further any liposomal drug or imaging agent from any leaked or free agent. The isolated liposomes were assayed for phospholipids (PL), doxorubicin (DOX), and Gd³⁺, and the change in Dox-to-PL or Gd³⁺-to-PL from the initial sample was calculated as a measure of the drug and/or detectable marker leakage from the liposomes. Doxorubicin was determined by spectrophotometric analysis in 70 % acid isopropanol at 498 nm and the phospholipid was determined by phosphate analysis using a modification of the method of Bartlett (1959). The Gd concentration was determined by NMR based on the effect of Gd on the proton relaxivity following dissolution of the liposomes in the presence of detergent (1 % Triton X-100) at 60 °C for 5 min. Similar to the results observed before, the stability of doxorubicin encapsulation is very good, with greater than 80 % of the doxorubicin remaining associated with the liposome after 48 h (see Fig. 17). The figure shows plasma stability of doxorubicin encapsulation in liposomes loaded using either Gd-DTPA or Fe(III)citrate as a trapping agent for the drug. The change in doxorubicin to phospholipid ratio and be directly correlated with the loss of doxorubicin from the liposome. The liposome samples were incubated with 50 % human plasma in a microdialysis assay at 37 °C as previously described.

The change in the Gd-to-phospholipid over time followed a similar trend. The amount of Gd that remained encapsulated after 72 h incubation was close to that of doxorubicin (75 % retained). Thus, the dual drug-paramagnetic marker co-loaded liposomes according to the invention were not only active with regard to both components, but also stable against leakage of the components from the liposomes in the presence of human blood plasma.

Figure 18 shows plasma stability of gadolinium encapsulation in liposomes loaded using Gd-DTPA as a trapping agent for doxorubicin. The change in Gd-to-phospholipid ratio can be directly correlated with the loss of Gd from the liposome. The liposome samples were incubated with 50 % human plasma in a microdialysis assay at 37 °C.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

The following numbered statements form part of the description:
1. A liposome for remote sensing of endocytosis comprising a detectable marker whose signal is modulated upon endocytosis of the liposome into a cell of interest.
2. The liposome of statement 1, wherein the detectable marker is paramagnetic metal chelate or a paramagnetic metal chelate conjugated to a lipopolymer.
3. The liposome of statement 2, wherein the detectable marker is selected from the group consisting of gadolinium DTPA-BMA, gadolinium DTPA and gadolinium HP-DO3A.
4. The liposome of statement 2, wherein the liposome comprises a poorly water-permeable membrane.
5. The liposome of statement 1, wherein the detectable marker is a fluorescent marker and the liposome further comprises a fluorescent quencher.
6. The liposome of statement 1, wherein the detectable marker is a fluorescent marker and is present at a self-quenching concentration.
7. The liposome of statement 1 further comprising a cell-internalizable ligand.
8. The liposome of statement 7, wherein said cell-internalizable ligand is an antibody fragment.
9. The liposome of statement 8, wherein said liposome comprises the antibody covalently conjugated to a terminally derivatized PEG-phosphatidylethanolamine linker.
10. The liposome of statement 1, wherein the detectable marker is conjugated to a lipopolymer.
11. The liposome of statement 1, wherein the liposome comprises pH-sensitive lipopolymers.
12. A method for remote sensing of endocytosis of a liposome, which method comprises :
   (a) contacting a cell with a liposome comprising a detectable marker whose signal is modulated upon endocytosis of the liposome into a cell of interest under conditions in which endocytosis can occur and
   (b) detecting the signal of the detectable marker after endocytosis.
13. The method of statement 12, wherein the detectable marker is selected from the group consisting of gadolinium DTPA-BMA, gadolinium DTPA and gadolinium HP-DO3A.
14. The method of statement 12, wherein the liposome comprises a poorly water-permeable membrane.
15. The method of statement 12, wherein said detecting step comprises an increase in proton relaxivity by MRI method.
16. The method of statement 12, wherein the detectable marker is a fluorescent marker and the liposome further comprises a fluorescent quencher.
17. The method of statement 16, wherein said detecting step comprising detecting increased fluorescence activity by laser imaging methods.
18. The method of statement 12, wherein the detectable marker is a fluorescent marker and is present at a self-quenching concentration.
19. The method of statement 18, wherein said detecting step comprising detecting increased fluorescence activity by laser imaging methods.
20. The method of statement 12, wherein the liposome comprises a cell-internalizable ligand.
21. The method of statement 12, wherein the detectable marker is conjugated to a lipopolymer.
22. A liposome comprising:
   (a) a remotely detectable marker; and
   (b) a therapeutic agent.
23. The liposome of statement 22, wherein said remotely detectable marker is a detectable marker whose signal is modulated upon endocytosis of the liposome into a cell of interest.
24. The liposome of statement 22 wherein the remotely detectable marker is conjugated to a lipopolymer.
25. The liposome of statement 24, wherein said conjugated detectable marker is distearoylamino-PEG-(Lys-Gd-DOTA)₄.
26. The liposome of statement 22, wherein said therapeutic agent is an anti-cancer agent.
27. The liposome of statement 26, wherein said anti-cancer agent is doxorubicin.
28. The liposome of statement 22, wherein said therapeutic agent and said remotely detectable marker, together, are an ionically coupled pair.
29. The liposome of statement 28, wherein said therapeutic agent is a cation and said remotely detectable marker is an anion.
30. The liposome of statement 28, wherein said therapeutic agent is an anion and said remotely detectable marker is a cation
31. The liposome of statement 22 further comprising a cell-internalizable ligand.
32. The liposome of statement 31, wherein said cell-internalizable ligand is an antibody fragment.
33. The liposome of statement 32, wherein said liposome comprises the antibody fragment covalently conjugated to a terminally derivatized PEG-phosphatidylethanolamine linker.
34. The liposome of statement 32, wherein said antibody fragment is an anti-HER2 antibody fragment and the therapeutic agent is doxorubicin.
35. A method for non-invasive monitoring of a liposomal drug in a patient's body comprising:
   (a) administering a liposome comprising
      (i) a remotely detectable marker whose signal is modulated upon endocytosis of the liposome into a cell of interest, and
      (ii) a therapeutic agent and
   (b) detecting the signal of the remotely detectable marker.
36. The method of statement 35, wherein the detectable marker is paramagnetic gadolinium chelate comprising gadolinium DTPA-BMA and gadolinium HP-DO3A.
37. The method of statement 36, wherein the liposome comprises a poorly water-permeable membrane.
38. The method of statement 35, wherein said detecting step comprises an increase in proton relaxivity by MRI method.
39. The method of statement 35, wherein the detectable marker is a fluorescent marker and the liposome further comprises a fluorescent quencher.
40. The method of statement 39, wherein said detecting step comprising detecting increased fluorescence activity by laser imaging methods.
41. The method of statement 35, wherein the detectable marker is a fluorescent marker and is present at a self-quenching concentration.
42. The method of statement 41, wherein said detecting step comprising detecting increased fluorescence activity by laser imaging methods.
43. The method of statement 35, wherein the liposome comprises a cell-internalizable ligand.
44. The method of statement 35, wherein said therapeutic agent and said remotely detectable marker, together, are an ionically coupled pair.
45. The method of statement 44, wherein said therapeutic agent is a cation and said remotely detectable marker is an anion.
46. The method of statement 44, wherein said therapeutic agent is an anion and said remotely detectable marker is a cation.
47. The method of statement 35, wherein said therapeutic agent is an anti-cancer agent.
48. The method of statement 47, wherein said anti-cancer agent is doxorubicin.
49. The method of statement 35, wherein said remotely detectable marker is conjugated to a lipopolymer.

## Claims

1. A detection liposome comprising an antibody fragment specific for and binding to a cell surface marker molecule; and a remotely detectable marker capable of producing a signal, wherein said marker comprises a paramagnetic metal chelate or a radioactive ion;
wherein the liposome is for use in;
(i) a method for determining the presence of a diseased cell comprising the cell surface marker molecule, said method comprising
contacting said cell with the liposome; and
determining the presence of the diseased cell by measuring an increase in the level of the signal due to binding of the antibody fragments to the cell surface marker molecules; or
(ii) a method for determining the presence of a disease condition **characterized by** at least one cell surface marker molecule in a patient, said method comprising;
contacting cells which are suspected to be diseased with the detection liposome and
measuring of the accumulation of the detection liposomes at the diseased site by measuring the level of signal produced which signal correlates to the level of binding of the detection liposome to said cell surface marker molecule, or
(iii) a method for determining the presence and treatability of a disease condition in a patient, wherein said disease is **characterized by** at least one cell surface marker molecule; said method comprising;
determining the presence of the disease by measuring the presence of accumulation of detection liposomes on a cell having the cell surface marker molecule; wherein accumulation of the detection liposomes is predictive of treatability with a therapeutic liposome comprising a therapeutic agent for treating said disease and the antibody fragment specific for and binding to said cell surface marker molecule; or
(iv) a method for remote sensing of endocytosis of a liposome, which method comprising (a) contacting a cell with the detection liposome under conditions in which endocytosis can occur; wherein the signal of the detectable marker is modulated upon endocytosis of the liposome into the cell; and (b) detecting the signal of the detectable marker after endocytosis; or
(v) a method for non-invasive monitoring of a liposomal drug in a patient's body comprising: (a) administering to said patient the detection liposome wherein the detection liposome further comprises a therapeutic agent and the signal of remotely detectable marker is modulated upon endocytosis of the liposome into a cell of interest, and (b) detecting the signal of the remotely detectable marker.

2. The liposome of claim 1, which is capable of endocytosis into the diseased cell or cell having the cell surface marker molecule and the liposomal membrane is capable of degradation upon endocytosis of the liposome.

3. The liposome of claim 2, wherein the detectable marker produces a modulated signal upon endocytosis of the liposome and liposomal degradation as compared to the signal produced prior to endocytosis of the liposome and liposomal degradation.

4. The liposome of claim 3, wherein the remotely detectable marker is a paramagnetic metal chelate.

5. The detection liposome of claim 1, wherein the remotely detectable marker is a radioactive ion.

6. The liposome of claim 2, wherein the remotely detectable marker is a radioactive ion and the presence of the diseased cell is determined by measuring the accumulation of the liposomes on or in the diseased cell.

7. The liposome of claim 5 or claim 6, wherein the liposome is capable of endocytosis into the diseased cell and the liposomal membrane is capable of degradation upon endocytosis of the liposome.

8. A liposome for use in treating a patient with a disease condition **characterized by** at least one cell surface marker molecule or improving treatment of a patient with a disease condition **characterized by** at least one cell surface marker molecule, which treatment comprises confirming that the condition is compatible with endocytotic delivery of a therapeutic agent for treating the disease, and administering a therapeutic amount of the therapeutic agent via a liposome to said patient.
